# EUROPEAN PATENT APPLICATION

(11) **EP 4 678 713 A1**
(43) Date of publication of application: **14.01.2026**
(21) Application number: 24767197.7
(22) Date of filing: 06.03.2024
(51) Int. Cl.: C09K 5/14, A61B 5/055, F25B 9/00, F25B 9/12, F28D 20/00, G01N 24/00, H01F 1/01, H01F 6/04

(54) **COLD STORAGE MATERIAL, COLD STORAGE MATERIAL PARTICLES, GRANULATED PARTICLES, COLD STORAGE DEVICE, REFRIGERATOR, CRYOPUMP, SUPERCONDUCTING MAGNET, NUCLEAR MAGNETIC RESONANCE IMAGING APPARATUS, NUCLEAR MAGNETIC RESONANCE APPARATUS, MAGNETIC FIELD APPLICATION-TYPE SINGLE CRYSTAL PULLING APPARATUS, HELIUM RECONDENSATION DEVICE, AND DILUTION REFRIGERATOR**

(30) Priority: 08.03.2023 JP 2023035607
(71) Applicant: Niterra Materials Co., Ltd., Yokohama-shi, Kanagawa 235-0032 (JP)
(72) Inventor: HINO, Takashi, Yokohama-shi, Kanagawa 235-0032 (JP); USUI, Daichi, Yokohama-shi, Kanagawa 235-0032 (JP); HIRAMATSU, Ryosuke, Yokohama-shi Kanagawa 235-0032 (JP)
(74) Representative: Henkel & Partner mbB
(86) International application number: PCT/JP2024/008624
(87) International publication number: WO 2024/185831

(57) **Abstract**

[PROBLEM] Provided is a cold storage material having a high volumetric specific heat and a high thermal conductivity

[SOLUTION] A cold storage material according to an embodiment contains a rare earth aluminum oxide containing at least one rare earth element selected from the group consisting of Y, La, Ce, Pr, Nd, Sm, Eu, Gd, Tb, Dy, Ho, Er, Tm, Yb, and Lu, in which a Group 1 element is contained in an amount of 0.001 atom% or more and 10 atom% or less, and a maximum volumetric specific heat value in a temperature range of 2 K or more and 10 K or less is 0.5 J/(cm³·K) or more.

## Description

### TECHNICAL FIELD

Embodiments described herein relate generally to a cold storage material, a cold storage material particle, a granulated particle, a cold storage device, a refrigerator, a cryopump, a superconducting magnet, a nuclear magnetic resonance imaging apparatus, a nuclear magnetic resonance apparatus, a magnetic field application type single crystal pulling apparatus, a helium re-condensing device, and a dilution refrigerator.

### BACKGROUND ART

In recent years, superconducting technologies have been remarkably developed, and it is necessary to develop compact and high-performance cryogenic refrigerators as the fields to which the superconducting technologies are applied have expanded. Such cryogenic refrigerators are required to be lightweight, compact, and highly thermally efficient, and have been put into practical use in various application fields.

In a cryogenic refrigerator, a plurality of cold storage materials are accommodated in a cold storage device. For example, cold is generated by heat exchange between the cold storage material and helium gas passing through the cold storage device. For example, in a cryopump or the like used in a superconducting MRI apparatus, a semiconductor manufacturing device, or the like, a refrigerator using a refrigeration cycle of a Gifford-McMahon (GM) type, a Stirling type, or a pulse tube type is used.

In addition, a high-performance refrigerator is also required for a magnetically levitated train in order to generate a magnetic force using a superconducting magnet. Furthermore, in recent years, a high-performance refrigerator has also been used in a superconducting magnetic energy storage (SMES), a magnetic field application type single crystal pulling apparatus for producing high-quality silicon wafers, or the like. Pulse tube refrigerators, which are expected to be even more reliable, are also being actively developed and put into practical use.

In addition, liquid helium used in the superconducting magnet, the MRI apparatus, or the like as described above evaporates, causing a liquid helium supplying issue. In recent years, the helium depletion problem has become serious, and it is difficult to obtain helium, affecting the industry.

In order to reduce the consumption of liquid helium and reduce the burden of maintenance such as replenishment, helium re-condensing devices for re-condensing evaporated helium have been put into practical use, and the demand for the helium re-condensing devices has increased. The helium re-condensing device also uses a GM type refrigerator or a pulse tube type refrigerator that cools helium to a 4K-level temperature to liquefy the helium.

In such a refrigerator, a working medium such as compressed helium (He) gas flows in one direction in a cold storage device accommodating a cold storage material, and thermal energy thereof is supplied to the cold storage material. Then, the expanded working medium receives thermal energy from the cold storage material while flowing in the cold storage device in the opposite direction. As the recuperation effect is improved through such a process, thermal efficiency in the working medium cycle is improved, thereby achieving a lower temperature.

Here, as the specific heat per unit volume of the cold storage material filled in the cold storage device is higher, the thermal energy that can be stored in the cold storage material increases, thereby improving the refrigeration capacity of the refrigerator. Therefore, it is desirable to fill a cold storage material having a high specific heat at a low temperature on a low-temperature side of the cold storage device and fill a cold storage material having a high specific heat at a high temperature on a high-temperature side of the cold storage device.

A magnetic cold storage material has a high volumetric specific heat in a specific temperature range depending on its composition. Therefore, by combining a magnetic cold storage material having a different composition exhibiting a high volumetric specific heat in a target temperature range, the cold storage capacity is enhanced, and the refrigeration capacity of the refrigerator is improved.

In addition, as the thermal conductivity and the heat transfer coefficient of the cold storage material filled in the cold storage device are higher, the thermal energy transfer efficiency is improved, and the efficiency of the refrigerator is improved.

In a conventional refrigerator, 4 K refrigeration has been achieved by combining metal cold storage material particles such as lead (Pb), bismuth (Bi), or tin (Sn) on a high-temperature side with metal-based magnetic cold storage material particles such as Er₃Ni, ErNi, or HoCu₂ on a low-temperature side of 20 K or less.

In recent years, it has been attempted to improve the refrigeration capacity of the refrigerator by substituting some on the low-temperature side of the metal-based magnetic cold storage material particles filled on the low-temperature side of the refrigerator with ceramic magnetic cold storage material particles having a high specific heat in a temperature range of 2 K to 10 K, such as Gd₂O₂S, Tb₂O₂S, Dy₂O₂S, Ho₂O₂S, and GdAlO₃.

As the application of the above-described refrigerators to various cooling systems is being considered, there is a need to stably cool larger objects to be cooled, and accordingly, there is a demand for refrigerators with even greater improvements in their cooling capacity.

### CITATION LIST

### PATENT LITERATURE

Patent Literature 1: JP 2003-73661 A
Patent Literature 2: JP 2003-213252 A
Patent Literature 3: WO 2018/025581 A1
Patent Literature 4: JP 5010071 B2

### SUMMARY OF THE INVENTION

### PROBLEM TO BE SOLVED BY THE INVENTION

An object of the present invention is to provide a cold storage material having a high volumetric specific heat and a high thermal conductivity.

### MEANS FOR SOLVING PROBLEM

A cold storage material according to an embodiment comprising: a rare earth aluminum oxide containing at least one rare earth element selected from the group consisting of Y, La, Ce, Pr, Nd, Sm, Eu, Gd, Tb, Dy, Ho, Er, Tm, Yb, and Lu; and a Group 1 element in an amount of 0.001 atom% or more and 10 atom% or less, wherein a maximum volumetric specific heat value in a temperature range of 2 K or more and 10 K or less is 0.5 J/(cm³·K) or more.

### BRIEF DESCRIPTION OF THE DRAWINGS

FIG. 1 is a schematic cross-sectional view illustrating a cold storage material particle according to a second embodiment and a configuration of a main part of a refrigerator according to a fourth embodiment.
FIG. 2 is a cross-sectional view illustrating a schematic configuration of a cryopump according to a fifth embodiment.
FIG. 3 is a perspective view illustrating a schematic configuration of a superconducting magnet according to a sixth embodiment.
FIG. 4 is a cross-sectional view illustrating a schematic configuration of a nuclear magnetic resonance imaging apparatus according to a seventh embodiment.
FIG. 5 is a cross-sectional view illustrating a schematic configuration of a nuclear magnetic resonance apparatus according to an eighth embodiment.
FIG. 6 is a perspective view illustrating a schematic configuration of a magnetic field application type single crystal pulling apparatus according to a ninth embodiment.
FIG. 7 is a schematic diagram illustrating a schematic configuration of a helium re-condensing device according to a tenth embodiment.
FIG. 8 is a schematic diagram illustrating a schematic configuration of a dilution refrigerator according to an eleventh embodiment.

### EMDODIMENT(S) FOR CARRYING OUT THE INVENTION

Hereinafter, embodiments of the present invention will be described with reference to the drawings. In the following description, the same or similar members and the like will be denoted by the same reference signs, and the description of the members and the like once described may be appropriately omitted.

In the present specification, a cryogenic temperature means, for example, a temperature range in which a superconducting phenomenon can be industrially useful. The cryogenic temperature is, for example, in a temperature range of 20 K or less.

### (First Embodiment)

A cold storage material according to a first embodiment contains: a rare earth aluminum oxide containing at least one rare earth element selected from the group consisting of yttrium (Y), lanthanum (La), cerium (Ce), praseodymium (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), and lutetium (Lu); and 0.001 atom% or more and 10 atom% or less of a Group 1 element, and a maximum volumetric specific heat value in a temperature range of 2 K or more and 10 K or less of the cold storage material is 0.5 J/(cm³·K) or more.

The cold storage material according to the first embodiment has a volumetric specific heat of 0.5 J/(cm³·K) or more, for example, in a temperature range of 2.5 K or more and 10 K or less. In addition, the cold storage material according to the first embodiment has a volumetric specific heat of 0.55 J/(cm³·K) or more, for example, in a temperature range of 2 K or more and 8 K or less. In addition, the cold storage material according to the first embodiment has a volumetric specific heat of 0.6 J/(cm³·K) or more, for example, in a temperature range of 3 K or more and 7 K or less.

The rare earth aluminum oxide contained in the cold storage material according to the first embodiment is represented by, for example, the general formula RxAl₂₋ₓO₃ (where R denotes at least one rare earth element selected from the group consisting of Y, La, Ce, Pr, Nd, Sm, Eu, Gd, Tb, Dy, Ho, Er, Tm, Yb, and Lu, and X is 0.3≤ X≤ 1.5). In the rare earth aluminum oxide represented by the above general formula, the maximum volumetric specific heat value and the temperature indicating the maximum volumetric specific heat value vary depending on the selected rare earth element. Therefore, the specific heat characteristic can be adjusted by appropriately adjusting the proportion of the rare earth element.

The rare earth element is, for example, at least one rare earth element selected from the group consisting of Gd, Tb, Dy, Ho, and Er. The rare earth aluminum oxide contained in the cold storage material according to the first embodiment may contain, for example, two or more kinds of rare earth elements.

The rare earth aluminum oxide is, for example, crystalline.

The cold storage material according to the first embodiment contains a rare earth aluminum oxide as a main component. Among the substances contained in the cold storage material according to the first embodiment, for example, the rare earth aluminum oxide has the largest volume ratio. Among the substances contained in the cold storage material according to the first embodiment, for example, the rare earth aluminum oxide has a volume ratio of 80% or more. The volume ratio of the rare earth aluminum oxide can be determined, for example, by performing area analysis of the constituent elements of the cold storage material using energy dispersive X-ray spectroscopy.

Among the substances contained in the cold storage material according to the first embodiment, for example, the rare earth aluminum oxide has the largest molar ratio. Among the substances contained in the cold storage material according to the first embodiment, for example, the rare earth aluminum oxide has a molar ratio of 80% or more. The molar ratio of the rare earth aluminum oxide can be determined, for example, using a powder X-ray diffraction method.

The cold storage material according to the first embodiment contains a Group 1 element in a total amount of 0.001 atom% or more and 10 atom% or less. The Group 1 element is at least one element selected from the group consisting of lithium (Li), sodium (Na), potassium (K), rubidium (Rb), cesium (Cs), and francium (Fr). The Group 1 element is, for example, at least one element selected from the group consisting of Li, Na, and K. The cold storage material may contain, for example, two or more kinds of Group 1 elements.

The Group 1 element contained in the cold storage material exists, for example, in crystals of the rare earth aluminum oxide. The Group 1 element contained in the cold storage material exists, for example, at crystal grain boundaries of the rare earth aluminum oxide. The Group 1 element contained in the cold storage material exists, for example, on inner wall surfaces of voids existing in the cold storage material. The Group 1 element contained in the cold storage material exists, for example, in crystal grains of the rare earth aluminum oxide. The Group 1 element contained in the cold storage material exists, for example, in crystal grains other than the rare earth aluminum oxide contained in the cold storage material.

The cold storage material according to the first embodiment contains a Group 2 element in a total amount of 0 atom% or more and 10 atom% or less, for example, in addition to the Group 1 element. In addition, the cold storage material according to the first embodiment contains a Group 2 element, for example, in a total amount of 0.001 atom% or more and 10 atom% or less, for example, in addition to the Group 1 element.

The Group 2 element is at least one element selected from the group consisting of beryllium (Be), magnesium (Mg), calcium (Ca), strontium (Sr), barium (Ba), and radium (Ra). The Group 2 element is, for example, at least one element selected from the group consisting of magnesium (Mg), calcium (Ca), strontium (Sr), and barium (Ba).

The cold storage material may contain, for example, two or more kinds of Group 2 elements. The cold storage material does not need to contain a Group 2 element.

The Group 2 element contained in the cold storage material exists, for example, in crystals of the rare earth aluminum oxide. The Group 2 element contained in the cold storage material exists, for example, at crystal grain boundaries of the rare earth aluminum oxide. The Group 2 element contained in the cold storage material exists, for example, on inner wall surfaces of voids existing in the cold storage material. The Group 2 element contained in the cold storage material exists, for example, in crystal grains of the rare earth aluminum oxide. The Group 2 element contained in the cold storage material exists, for example, in crystal grains other than the rare earth aluminum oxide contained in the cold storage material.

The cold storage material according to the first embodiment contains, for example, a substance derived from a sintering aid used in producing the cold storage material. The sintering aid is, for example, a magnesium oxide, an yttrium oxide, a zirconium oxide, or a boron oxide.

The cold storage material according to the first embodiment contains, for example, 0.01 atom% or more and 20 atom% or less of at least one element selected from the group consisting of iron (Fe), copper (Cu), nickel (Ni), cobalt (Co), zirconium (Zr), and boron (B). The at least one element selected from the group consisting of Fe, Cu, Ni, Co, Zr, and B is, for example, an element derived from a sintering aid.

Note that the detection of the element contained in the cold storage material according to the first embodiment and the measurement of the atomic concentration of the element can also be performed, for example, by dissolving the cold storage material in a liquid, using inductively coupled plasma atomic emission spectroscopy (ICP-AES). It is also possible to use energy dispersive X-ray spectroscopy (EDX) or wavelength dispersive X-ray spectroscopy (WDX).

The crystal structure of the rare earth aluminum oxide contained in the cold storage material according to the first embodiment is, for example, of a GdFeO₃ type, and its space group is Pnma. The crystal structure can be confirmed by powder X-ray diffraction measurement, observation of an electron backscatter diffraction image using a scanning electron microscope, transmission electron microscopy, or the like.

The method for producing the cold storage material according to the first embodiment is not particularly limited, but for example, the cold storage material according to the first embodiment can be produced by mixing raw material powders using a ball mill or the like to prepare a raw material mixture, and molding and sintering the obtained raw material mixture. As the raw material powders, a rare earth oxide and an aluminum oxide can be used. The type and the proportion of the rare earth oxide are adjusted in accordance with the target composition of the cold storage material.

By using a carbonate containing a Group 1 element, an oxide containing a Group 1 element, a nitride containing a Group 1 element, or a carbide containing a Group 1 element in the raw material powder, the Group 1 element can be contained in the cold storage material. By using a carbonate containing a Group 2 element, an oxide containing a Group 2 element, a nitride containing a Group 2 element, or a carbide containing a Group 2 element in the raw material powder, the Group 2 element can be contained in the cold storage material.

The raw material mixture may contain a sintering aid. The sintering aid is, for example, an yttrium oxide, a zirconium oxide, or a boron oxide.

The heat treatment for sintering the molded body is performed, for example, in a pressurized inert gas atmosphere. The heat treatment temperature is, for example, 1000°C or more and 2000°C or less. The heat treatment temperature is, for example, 1100°C or more and 1700°C or less. The heat treatment time is, for example, 1 hour or more and 48 hours or less.

The cold storage material according to the first embodiment may be, for example, a sintered body of cold storage material particles made of a first cold storage material.

Next, the function and effect of the cold storage material according to the first embodiment will be described.

In a cryogenic refrigerator used for cooling a superconducting device or the like, the cold storage material is accommodated in a cold storage device. For example, cold is generated by heat exchange between the cold storage material and helium gas passing through the cold storage device. In order to improve the refrigeration capacity of the refrigerator, the cold storage material accommodated in the cold storage device is required to have excellent characteristics such as a high volumetric specific heat and a high thermal conductivity.

The upper limit of the volumetric specific heat is limited by the composition of the substance. Therefore, it is difficult to significantly improve the volumetric specific heat. On the other hand, the thermal conductivity can be improved by improving crystallinity and reducing the number of voids.

The maximum volumetric specific heat value of the cold storage material according to the first embodiment in a temperature range of 2 K or more and 10 K or less is 0.5 J/(cm³·K) or more. As a result, the cold storage material according to the first embodiment has a high volumetric specific heat.

In addition, the cold storage material according to the first embodiment has a volumetric specific heat of 0.5 J/(cm³·K) or more, for example, in a temperature range of 2.5 K or more and 10 K or less. In addition, the cold storage material according to the first embodiment has a volumetric specific heat of 0.55 J/(cm³·K) or more, for example, in a temperature range of 2 K or more and 8 K or less. In addition, the cold storage material according to the first embodiment has a volumetric specific heat of 0.6 J/(cm³·K) or more, for example, in a temperature range of 3 K or more and 7 K or less.

As described above, since the cold storage material according to the first embodiment has a high volumetric specific heat, the cold storage device accommodating the cold storage material according to the first embodiment has high cold storage performance. The refrigerator including the cold storage device accommodating the cold storage material according to the first embodiment exhibits high refrigeration capacity.

In addition, the cold storage material according to the first embodiment contains a Group 1 element in an amount of 0.001 atom% or more and 10 atom% or less in terms of atomic concentration. The Group 1 element acts to promote the sintering of the molded body and reduce the number of voids in the obtained sintered body in the sintering process when the cold storage material is produced. Therefore, the cold storage material according to the first embodiment has a high degree of sintering and a high thermal conductivity.

In order to sufficiently obtain characteristics required for the cold storage material such as thermal conductivity and volumetric specific heat, a sufficient sintering temperature and a sufficient sintering time are required in the sintering process. The cold storage material according to the first embodiment makes it possible to lower the sintering temperature required for sintering and to reduce the sintering time by the sintering promoting action of the Group 1 element. Therefore, it is possible to reduce the cost for producing the cold storage material, providing an inexpensive cold storage material.

When the atomic concentration of the Group 1 element in the cold storage material is 0.001 atom% or more, the degree of sintering increases, and the number of extremely minute voids decreases. Therefore, it is possible to increase the thermal conductivity of the cold storage material.

When the atomic concentration of the Group 1 element in the cold storage material exceeds 10 atom%, an oxide containing a rare earth and the Group 1 element is generated, lowering the volumetric specific heat and lowering the thermal conductivity. When the atomic concentration of the Group 1 element in the cold storage material is 10 atom% or less, the volumetric specific heat is high and the thermal conductivity can be increased, thereby improving the refrigeration capacity of the refrigerator accommodating the cold storage material according to the first embodiment.

The Group 1 element contained in the cold storage material according to the first embodiment is, for example, at least one element selected from the group consisting of Li, Na, K, Rb, Cs, and Fr. In addition, the Group 1 element is preferably at least one element selected from the group consisting of Li, Na, and K. In addition, for example, two or more kinds of Group 1 elements among the Group 1 elements may be contained.

By using a carbonate, an oxide, a nitride, or a carbide containing a Group 1 element in the raw material powder, a cold storage material containing the Group 1 element can be produced. The concentration of the Group 1 element contained in the cold storage material is adjusted by adjusting the amount of carbonate, oxide, nitride, or carbide containing the Group 1 element.

The cold storage material according to the first embodiment contains a Group 2 element, for example, in a total amount of 0 atom% or more and 10 atom% or less in terms of atomic concentration. In addition, the cold storage material according to the first embodiment contains a Group 2 element, for example, in a total amount of 0.001 atom% or more and 10 atom% or less in terms of atomic concentration. The Group 2 element is at least one element selected from the group consisting of Be, Mg, Ca, Sr, Ba, and Ra. The atomic concentration of the Group 2 element is, for example, 0.001 atom% or more and 5 atom% or less in total. The cold storage material may contain, for example, two or more kinds of Group 2 elements.

In the cold storage material according to the first embodiment, the Group 2 element is contained in an amount of 0.001 atom% or more in addition to the Group 1 element, thereby further improving the sinterability and reducing the number of extremely minute voids. This further improves the thermal conductivity. The Group 2 element does not exhibit specific heat characteristics. Therefore, when the Group 2 element is contained in a total amount of more than 10 atom%, the volumetric specific heat of the cold storage material decreases, resulting in a decrease in cold storage performance of the cold storage device and a decrease in refrigeration capacity of the refrigerator.

By using a carbonate, an oxide, a nitride, or a carbide containing a Group 2 element in the raw material powder, a cold storage material containing the Group 2 element can be produced. The concentration of the Group 2 element contained in the cold storage material is adjusted by adjusting the amount of carbonate, oxide, nitride, or carbide containing the Group 2 element.

When the cold storage material according to the first embodiment is produced, by adding a sintering aid in an amount of 0.01 atom% or more as a metal or a metalloid element constituting the sintering aid in addition to the Group 1 element, the sinterability is further improved, and the number of extremely minute voids is reduced. This further improves the thermal conductivity. The metal or metalloid element constituting the sintering aid does not exhibit specific heat characteristics. Therefore, when the metal or metalloid element constituting the sintering aid is added in an amount of more than 20 atom%, the volumetric specific heat of the cold storage material decreases, resulting in a decrease in cold storage performance of the cold storage device and a decrease in refrigeration capacity of the refrigerator.

The cold storage material according to the first embodiment preferably contains at least one element selected from the group consisting of iron (Fe), copper (Cu), nickel (Ni), cobalt (Co), zirconium (Zr), and boron (B) in an amount of 0.01 atom% or more and 20 atom% or less. The at least one element selected from the group consisting of Fe, Cu, Ni, Co, Zr, and B is, for example, an element derived from a sintering aid. The at least one element selected from the group consisting of Fe, Cu, Ni, Co, Zr, and B is, for example, an example of a metal or metalloid element constituting the sintering aid.

As described above, according to the first embodiment, it is possible to realize a cold storage material having excellent characteristics such as a high volumetric specific heat and a high thermal conductivity.

### (Second Embodiment)

The cold storage material particle according to the second embodiment is made of the cold storage material according to the first embodiment, and has a particle diameter of 50 µm or more and 3 mm or less. The cold storage material particle has an aspect ratio of, for example, 1 or more and 5 or less. The aspect ratio of the cold storage material particle is a ratio of a long diameter to a short diameter of the cold storage material particle. The shape of the cold storage material particle is, for example, spherical.

Hereinafter, some of the explanations overlapping with those in the first embodiment may be omitted.

The particle diameter of the cold storage material particle is an equivalent circle diameter. The equivalent circle diameter is a diameter of a perfect circle corresponding to an area of a figure observed in an image such as an optical microscope image or a scanning electron microscope image (SEM image). The particle diameter of the cold storage material particle can be obtained, for example, by image analysis on an optical microscope image or an SEM image.

The method for producing the cold storage material particle according to the second embodiment is not particularly limited, but for example, the cold storage material particle according to the second embodiment can be produced by mixing raw material powders using a ball mill or the like to prepare a raw material mixture, molding (granulating) the obtained raw material mixture into a granule by a rolling granulation method, a stirring granulation method, an extrusion method, a spraying method, a press molding method, or the like, and then sintering the obtained granular molded body.

The obtained granular molded body is referred to as a granulated particle.

In the granulation method, a binder is added, so that the raw material powders adhere to each other, thereby improving the strength of the granulated particle. The binder is, for example, polyvinyl alcohol, polyvinyl butyral, carboxymethyl cellulose, an acrylic resin, or polyethylene glycol. The binder is added in an amount of, for example, 0.01 wt% or more and 20 wt% or less.

As the raw material powders, a rare earth oxide and an aluminum oxide can be used. The type and the proportion of the rare earth oxide are adjusted in accordance with the target composition of the cold storage material particle.

By using a carbonate containing a Group 1 element, an oxide containing a Group 1 element, a nitride containing a Group 1 element, or a carbide containing a Group 1 element in the raw material powder, a cold storage material particle containing the Group 1 element can be produced. In addition, by using a carbonate containing a Group 2 element, an oxide containing a Group 2 element, a nitride containing a Group 2 element, or a carbide containing a Group 2 element in the raw material powder, a cold storage material particle containing the Group 2 element can be produced.

By adding a sintering aid to the raw material powder, a cold storage material particle containing the sintering aid can be produced. The sintering aid may react with the rare earth oxide or the aluminum oxide, which is a raw material, thereby producing an oxide phase containing a rare earth element, aluminum, and a metal or a metalloid element constituting the sintering aid.

In addition, a slurry prepared by adding raw material powders to an alginate aqueous solution and mixing the raw material powders with the alginate aqueous solution may be dropped into a gelling solution, and the slurry may be gelled to granulate the slurry in a granular form. This is a method in which particles are granulated by causing gelation through a crosslinking reaction by polyvalent metal ions contained in the gelling solution. Therefore, the strength of the granulated particle, that is, the gelation strength, changes depending on the amount of alginate contained in the particle.

The amount of alginate contained in the particle can be changed depending on the concentration of alginate contained in the alginate aqueous solution or the ratio between the alginate aqueous solution and the raw material powders. The slurry can be dropped into the gelling solution, for example, using a dropper, a burette, a pipette, a syringe, a dispenser, an inkjet, or the like. Hereinafter, the foregoing particle granulation method will be referred to as an alginate gel method.

In the alginate gel method, the particle diameter and the aspect ratio of the particle can be changed by adjusting the viscosity of the slurry, the diameter of the discharge port at the time of dropping the slurry, or the distance between the tip of the discharge port and the liquid level of the gelling solution. The diameter of the discharge port is, for example, 50 µm or more and 3000 µm or less. The viscosity of the slurry is, for example, 0.1 mPa·s or more and 1000000 mPa·s or less. The distance between the tip of the discharge port and the liquid level of the gelling solution is, for example, 0.1 mm or more and 1000 mm or less.

In a case where the dispenser is used for dispensing the slurry, any one of an air pulse type dispenser, a plunger type dispenser, and a piezo type dispenser may be used as the device.

The inkjet is largely classified as a continuous type inkjet or an on-demand type inkjet as an ejection method, but any type of ejection method may be used. Further, the on-demand type inkjet is classified as a piezo type inkjet, a thermal type inkjet, or a valve type inkjet, but any one of the three types of inkjets may be used.

The slurry dropped into the gelling solution by a dropper, a burette, a pipette, a syringe, a dispenser, an inkjet, or the like is gelled by being held in the gelling solution. By gelling the slurry, a granulated particle containing the raw material powders of the cold storage material is formed.

The time for which the slurry is held in the gelling solution is, for example, 10 minutes or more and 48 hours or less. If the gelation time is short, gelation does not sufficiently proceed, and the strength of the granulated particle is smaller than the strength expected from the amount of alginate.

The alginate aqueous solution used in the alginate gel method is, for example, a sodium alginate aqueous solution, an ammonium alginate aqueous solution, or a potassium alginate aqueous solution. By using a sodium alginate aqueous solution or a potassium alginate aqueous solution containing a Group 1 element, sodium or potassium can be contained in the cold storage material particle. By using a mixed aqueous solution of a sodium alginate aqueous solution and a potassium alginate aqueous solution in the slurry, sodium and potassium can be simultaneously contained in the cold storage material particle.

By adjusting the concentration of the alginate containing the Group 1 element, the concentration of the Group 1 element contained in the particle is adjusted. The alginate has a concentration of, for example, 0.01 wt% or more and 5 wt% or less as an alginate aqueous solution. When the concentration of the alginate aqueous solution is lower than 0.01 wt%, a gel having sufficient strength cannot be generated, and particles cannot be obtained.

As the gelling solution, for example, a calcium lactate aqueous solution, a calcium chloride aqueous solution, a manganese (II) chloride aqueous solution, a magnesium sulfate aqueous solution, a beryllium sulfate aqueous solution, a strontium nitrate aqueous solution, a barium chloride aqueous solution, a barium hydroxide aqueous solution, an aluminum chloride aqueous solution, an aluminum nitrate aqueous solution, an aluminum lactate aqueous solution, an iron (II) chloride aqueous solution, an iron (III) chloride aqueous solution, a copper (II) chloride aqueous solution, a nickel (II) chloride aqueous solution, or a cobalt (II) chloride aqueous solution can be used.

By using a calcium lactate aqueous solution, a calcium chloride aqueous solution, a magnesium sulfate aqueous solution, a beryllium sulfate aqueous solution, a strontium nitrate aqueous solution, a barium chloride aqueous solution, or a barium hydroxide aqueous solution as an aqueous solution containing a Group 2 element, which is a gelling solution, calcium, magnesium, beryllium, strontium, or barium can be contained in the cold storage material particle.

By using an iron (II) chloride aqueous solution, an iron (III) chloride aqueous solution, a copper (II) chloride aqueous solution, a nickel (II) chloride aqueous solution, or a cobalt (II) chloride aqueous solution as a gelling solution, iron, copper, nickel, or cobalt can be contained in the cold storage material particle as a sintering aid.

Since gelation is caused through a crosslinking reaction by polyvalent metal ions contained in the gelling solution, in a case where an aqueous solution containing a Group 1 element is used for the slurry and an aqueous solution containing a Group 2 element is used for the gelling solution, the amounts of the Group 1 element and the Group 2 element contained in the particle can be adjusted by adjusting a time for which the particle granulated by dropping the slurry into the gelling solution is immersed in the gelling solution.

By mixing at least two kinds of aqueous solutions containing different metal elements selected from the group consisting of a calcium lactate aqueous solution, a calcium chloride aqueous solution, a magnesium sulfate aqueous solution, a beryllium sulfate aqueous solution, a strontium nitrate aqueous solution, a barium chloride aqueous solution, and a barium hydroxide aqueous solution for use as a gelling solution, two or more kinds of Group 2 elements can be contained in the cold storage material particle.

The granulated particle has a particle diameter of, for example, 70 µm or more and 5 mm or less. The granulated particle has an aspect ratio of, for example, 1 or more and 5 or less.

By degreasing the granulated particles, a certain amount of organic components can be removed. If the granulated particles are insufficiently degreased and the organic components remain too much, the density of the sintered particles is low. This weakens the strength of the cold storage material particle, making it impossible to withstand use in a refrigerator.

If the granulated particles are degreased too much, the organic components that ensure strength disappears, and the strength of the granulated particles after being degreased decreases, causing a crack or a chip. The degreasing temperature is, for example, 400°C or more and 800°C or less, and the degreasing time is 30 minutes or more and 12 hours or less.

The heat treatment for sintering the granulated particles after being degreased is performed, for example, in a pressurized inert gas atmosphere. The heat treatment temperature is, for example, 1000°C or more and 2000°C or less. The heat treatment temperature is, for example, 1100°C or more and 1700°C or less. The heat treatment time is, for example, 1 hour or more and 48 hours or less.

Next, the function and effect of the cold storage material particle according to the second embodiment will be described.

The cold storage material particle according to the second embodiment is made from the cold storage material according to the first embodiment, and has a particle diameter of 50 µm or more and 3 mm or less. The cold storage material particle has an aspect ratio of, for example, 1 or more and 5 or less. The aspect ratio of the cold storage material particle is a ratio of a long diameter to a short diameter of the cold storage material particle. The shape of the cold storage material particle is, for example, spherical.

The maximum volumetric specific heat value of the cold storage material particle according to the second embodiment in a temperature range of 2 K or more and 10 K or less is 0.5 J/(cm³·K) or more. As a result, the cold storage material particle according to the second embodiment has a high volumetric specific heat. Since the cold storage material particle according to the second embodiment has a high volumetric specific heat, a cold storage device filled with the cold storage material particle according to the second embodiment has high cold storage performance. In addition, the refrigerator including the cold storage device filled with the cold storage material particle according to the second embodiment exhibits high refrigeration capacity.

In addition, the cold storage material particle according to the second embodiment contains a Group 1 element in an amount of 0.001 atom% or more and 10 atom% or less in terms of atomic concentration. The Group 1 element acts to promote the sintering of the cold storage material particle and reduce the number of voids contained in the cold storage material particle during the sintering of the cold storage material particle when the cold storage material particle is produced. Therefore, the cold storage material particle has a high degree of sintering and a high thermal conductivity.

In the cold storage material particle according to the second embodiment, a sintering aid is added in an amount of 0.01 atom% or more as a metal or a metalloid element constituting the sintering aid in addition to the Group 1 element, thereby further improving the sinterability and reducing the number of extremely minute voids. This further improves the thermal conductivity. The metal or metalloid element constituting the sintering aid does not exhibit specific heat characteristics. Therefore, when the metal or metalloid element constituting the sintering aid is added in an amount of more than 20 atom%, the volumetric specific heat of the cold storage material particle decreases, resulting in a decrease in cold storage performance of the cold storage device and a decrease in refrigeration capacity of the refrigerator.

In order to sufficiently obtain characteristics required for the cold storage material particle such as thermal conductivity and volumetric specific heat, a sufficient sintering temperature and a sufficient sintering time are required in the sintering process. The cold storage material particle according to the second embodiment makes it possible to lower the sintering temperature required for sintering and to reduce the sintering time by the sintering promoting action of the Group 1 element. Therefore, it is possible to reduce the cost for producing the cold storage material particle, providing an inexpensive cold storage material particle.

The cold storage material particle according to the second embodiment has a particle diameter of 50 µm or more and 3 mm or less. The particle diameter of the cold storage material particle is more preferably 1 mm or less, and still more preferably 500 µm or less.

When the particle diameter of the cold storage material particle is larger than the lower limit value, the packing density of the cold storage material particle in the cold storage device is low, resulting in a reduction in pressure loss of the working medium such as helium, and an improvement in refrigeration performance of the refrigerator. On the other hand, when the particle diameter of the cold storage material particle is smaller than the upper limit value, a distance from the surface of the cold storage material particle to the central portion of the particle is short, making it easy to convey heat transfer between the working medium and the cold storage material particle to the central portion of the cold storage material, resulting in an improvement in refrigeration performance of the refrigerator.

The aspect ratio of the cold storage material particle is preferably 1 or more and 5 or less, and more preferably 1 or more and 2 or less. When the aspect ratio of the cold storage material particle is smaller than the upper limit value, voids when cold storage material particles are filled in a cold storage device are uniform, improving the refrigeration performance of the refrigerator.

In the cold storage material particle according to the second embodiment, if the granulated particle after being degreased does not satisfy a certain level or more of strength, the granulated particle is cracked or chipped while being handled. When non-spherical cold storage material particles are filled in the refrigerator, the refrigerator performance decreases, and thus, cracked or chipped granulated particles are discarded as defective products. For this reason, it is desirable that the granulated particle has a certain level of strength or more to prevent a crack or a chip.

The strength of the granulated particle mainly depends on the amount of binder or the amount of alginate, but if the amount of these organic components is too large, sintering becomes difficult. On the other hand, the Group 1 element promotes the sintering of the cold storage material particle. When the granulated particle contains a Group 1 element in an amount of 0.001 atom% or more and 10 atom% or less in terms of atomic concentration and further contains a binder or a carbon component derived from alginate in an amount of 0.01 wt% or more and 20 wt% or less, both sinterability and high strength can be achieved by the effects of the two components.

The content of the carbon component is more preferably 10 wt% or less, and still more preferably 5 wt% or less. Even when the Group 1 element is contained in an amount of 0.001 atom% or more and 10 atom% or less in terms of atomic concentration, if the carbon component is contained in an amount of more than 20 wt%, it is difficult to appropriately remove the organic components, or the molding density after degreasing decreases significantly, and the sintering reaction does not proceed even at a high sintering temperature. For this reason, the strength of the sintered particle is remarkably low, making it difficult to recover the particle. If the carbon component is contained in an amount of less than 0.01 wt%, the amount of the binder or sodium alginate contained in the granulated particle is small, making the strength of the granulated particle weak, and causing a crack or a chip in the granulated particle when handled.

When the degreased granulated particle contains a Group 1 element in an amount of 0.001 atom% or more and 10 atom% or less in terms of atomic concentration, and further contains a binder or a carbon component derived from alginate in an amount of 0.001 wt% or more and 10 wt% or less, both sinterability and high strength can be achieved. The content of the carbon component is more preferably 5 wt% or less, and still more preferably 3 wt% or less. When the carbon component is contained in an amount of more than 10 wt%, the molding density is low, and accordingly, the density does not increase even after sintering, and the particles do not have enough strength to withstand use in a refrigerator. When it is attempted to improve the density after sintering by increasing the sintering temperature, particles adhere to each other, resulting in a significant decrease in aspect ratio.

In consideration of shrinkage caused by sintering, the particle diameter of the granulated particle is desirably 70 µm or more and 5 mm or less both before and after the granulated particle is degreased. The aspect ratio of the granulated particle is, for example, 1 or more and 5 or less both before and after the granulated particle is degreased.

As described above, according to the second embodiment, it is possible to realize a cold storage material particle having excellent characteristics such as a high volumetric specific heat and a high thermal conductivity.

### (Third Embodiment)

A cold storage device according to a third embodiment is a cold storage device filled with a plurality of cold storage material particles according to the second embodiment. In the cold storage device according to the third embodiment, for example, when a perimeter of a projection image of each of the plurality of filled cold storage material particles according to the second embodiment is denoted by L and an actual area of the projection image is denoted by A, the proportion of cold storage material particles having a circularity R of 0.5 or less, the circularity R being represented by 4πA/L², is 5% or less.

The circularity R can be obtained by performing image processing on the shapes of the plurality of cold storage material particles using an optical microscope. The cold storage material particle having a circularity R of 0.5 or less has a shape with irregularities on the surface or the like. When a plurality of cold storage material particles including such cold storage material particles in an amount of more than 5% are filled in the cold storage device, the porosity formed by the cold storage material particles in the cold storage device is uneven, and the filling property is unstable. Therefore, the cold storage performance deteriorates when the working medium flows in the cold storage device, or the cold storage material particles are moved or broken due to stress applied to the cold storage material particles at the time of filling the cold storage material particles or at the time of operating the refrigerator, generating fine particles, which causes voids to be clogged, thereby reducing the refrigeration performance and long-term reliability of the refrigerator. The proportion of cold storage material particles having a circularity R of 0.5 or less is preferably 2% or less, and more preferably 0%.

### (Fourth Embodiment)

A refrigerator according to a fourth embodiment is a refrigerator including a cold storage device filled with the cold storage material according to the first embodiment or the plurality of cold storage material particles according to the second embodiment. Hereinafter, some of the explanations overlapping with those in the first, second, and third embodiments will be omitted.

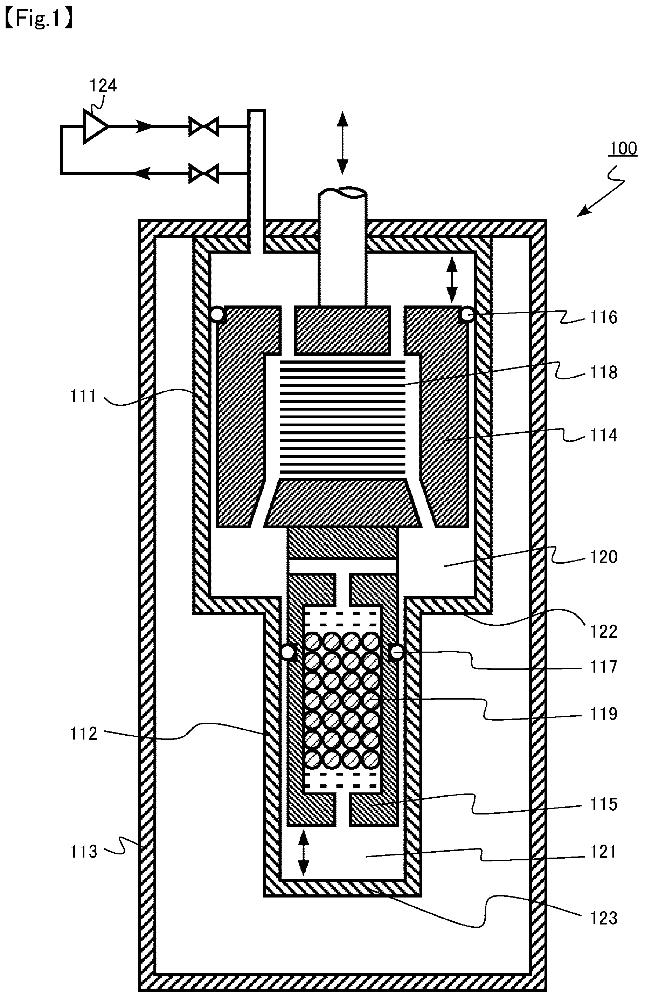

FIG. 1 is a schematic cross-sectional view illustrating a configuration of a main part of the refrigerator according to the fourth embodiment including the cold storage device according to the third embodiment filled with the plurality of cold storage material particles according to the second embodiment. The refrigerator according to the fourth embodiment is a two-stage cold storage cryogenic refrigerator 100 used for cooling a superconducting device or the like.

In the cold storage cryogenic refrigerator, cold is generated by heat exchange between the cold storage material or the cold storage material particles and helium gas passing through the cold storage device. For example, as a cryopump used in a superconducting MRI apparatus, a semiconductor manufacturing device, or the like, or a refrigerator mounted in a dilution refrigerator or the like, there is a refrigerator to which a refrigeration cycle of a Gifford-McMahon (GM) type, a Stirling type, a pulse tube type, or the like is applied.

The cold storage cryogenic refrigerator 100 includes a first cylinder 111, a second cylinder 112, a vacuum container 113, a first cold storage device 114, a second cold storage device 115, a first seal ring 116, a second seal ring 117, a first cold storage material 118, a second cold storage material 119, a first expansion chamber 120, a second expansion chamber 121, a first cooling stage 122, a second cooling stage 123, and a compressor 124.

The cold storage cryogenic refrigerator 100 includes a vacuum container 113 in which a large-diameter first cylinder 111 and a small-diameter second cylinder 112 coaxially connected to the first cylinder 111 are installed. The first cold storage device 114 is disposed in the first cylinder 111 so as to be reciprocable. The second cold storage device 115, which is an example of the cold storage device according to the third embodiment, is disposed in the second cylinder 112 so as to be reciprocable.

The first seal ring 116 is disposed between the first cylinder 111 and the first cold storage device 114. The second seal ring 117 is disposed between the second cylinder 112 and the second cold storage device 115.

The first cold storage material 118 such as a Cu mesh is accommodated in the first cold storage device 114. The second cold storage material 119 is accommodated in the second cold storage device 115.

Each of the first cold storage device 114 and the second cold storage device 115 has a working medium passage provided in a space in the first cold storage material 118 or the second cold storage material 119. The working medium is helium gas.

The first expansion chamber 120 is provided between the first cold storage device 114 and the second cold storage device 115. The second expansion chamber 121 is provided between the second cold storage device 115 and the distal end wall of the second cylinder 112. The first cooling stage 122 is provided at the bottom of the first expansion chamber 120. The second cooling stage 123 having a lower temperature than the first cooling stage 122 is formed at the bottom of the second expansion chamber 121.

A high-pressure working medium is supplied from the compressor 124 to the above-described two-stage cold storage cryogenic refrigerator 100. The supplied working medium passes through the first cold storage material 118 accommodated in the first cold storage device 114 and reaches the first expansion chamber 120. Then, the working medium passes through the second cold storage material 119 accommodated in the second cold storage device 115 and reaches the second expansion chamber 121.

At this time, the working medium is cooled by supplying thermal energy to the first cold storage material 118 and the second cold storage material 119. The working medium having passed through the first cold storage material 118 and the second cold storage material 119 expands in the first expansion chamber 120 and the second expansion chamber 121 to generate cold. Then, the first cooling stage 122 and the second cooling stage 123 are cooled.

The expanded working medium flows in the opposite direction through the first cold storage material 118 and the second cold storage material 119. The working medium is discharged after receiving thermal energy from the first cold storage material 118 and the second cold storage material 119. By improving the recuperation effect through such a process, the cold storage cryogenic refrigerator 100 is configured so that thermal efficiency in the working medium cycle is improved, thereby achieving a lower temperature.

As a cold storage device included in the refrigerator according to the fourth embodiment, the second cold storage device 115 accommodates the cold storage material according to the first embodiment as at least a part of the second cold storage material 119. In addition, the second cold storage device 115 may be filled with a plurality of cold storage material particles according to the second embodiment as at least some of the second cold storage material 119. With respect to the plurality of cold storage material particles according to the second embodiment, when a perimeter of a projection image of each of the cold storage material particles is denoted by L and an actual area of the projection image is denoted by A, it is preferable that the proportion of cold storage material particles having a circularity R of 0.5 or less, the circularity R being represented by 4πA/L², is 5% or less.

In the fourth embodiment, the cold storage device according to the third embodiment may include, for example, a plurality of cold storage material-filled layers of different types of cold storage materials. The different types of cold storage materials may be separated by a mesh. The mesh is, for example, a metal mesh. At least one of the plurality of cold storage material-filled layers is made of the cold storage material according to the first embodiment or the cold storage material particles according to the second embodiment. In the refrigerator according to the fourth embodiment, the cold storage material according to the first embodiment or the plurality of cold storage material particles according to the second embodiment are filled for example, on a low temperature side of the cold storage device.

The second cold storage device 115 may include, for example, two filled layers, in which the filled layer on the high-temperature side is filled with at least one type of cold storage material selected from the group consisting of Pb, Bi, Sn, Er3Ni, ErNi, and HoCu₂, and the filled layer on the low-temperature side is filled with the cold storage material according to the first embodiment or the cold storage material particles according to the second embodiment. In addition, the second cold storage device 115 may include, for example, three filled layers, in which the filled layer on the high temperature side is filled with at least one type of cold storage material selected from the group consisting of Pb, Bi, and Sn, the intermediate layer is filled with at least one type of cold storage material selected from the group consisting of Er₃Ni, ErNi, HoCu₂, Gd₂O₂S, Tb₂O₂S, Dy₂O₂S, and Ho₂O₂S, and the filled layer on the low temperature side is filled the cold storage material according to the first embodiment or the cold storage material particles according to the second embodiment. In a case where the second cold storage device 115 includes three filled layers, the filled layer on the high-temperature side may be filled with at least one type of cold storage material selected from the group consisting of Er₃Ni, ErNi, and HoCu₂, the intermediate layer may be filled with at least one type of cold storage material selected from the group consisting of Gd₂O₂S, Tb₂O₂S, Dy₂O₂S, and Ho₂O₂S, and the filled layer on the low-temperature side may be filled with the cold storage material according to the first embodiment or the cold storage material particles according to the second embodiment.

In order to improve the refrigeration capacity of the refrigerator, it is desirable to improve the specific heat per unit volume of the cold storage material and to improve the thermal conductivity of the cold storage material. The refrigerator according to the fourth embodiment includes a cold storage material or cold storage material particles which maintain a volumetric specific heat with improved thermal conductivity.

For example, by using the refrigerator according to the fourth embodiment in a magnetically levitated train, the long-term reliability of the magnetically levitated train can be improved.

As described above, according to the fourth embodiment, a refrigerator having excellent characteristics can be realized by using a cold storage material or cold storage material particles having excellent characteristics.

### (Fifth Embodiment)

A cryopump according to a fifth embodiment includes the refrigerator according to the fourth embodiment. Hereinafter, some of the explanations overlapping with those in the fourth embodiment will be omitted.

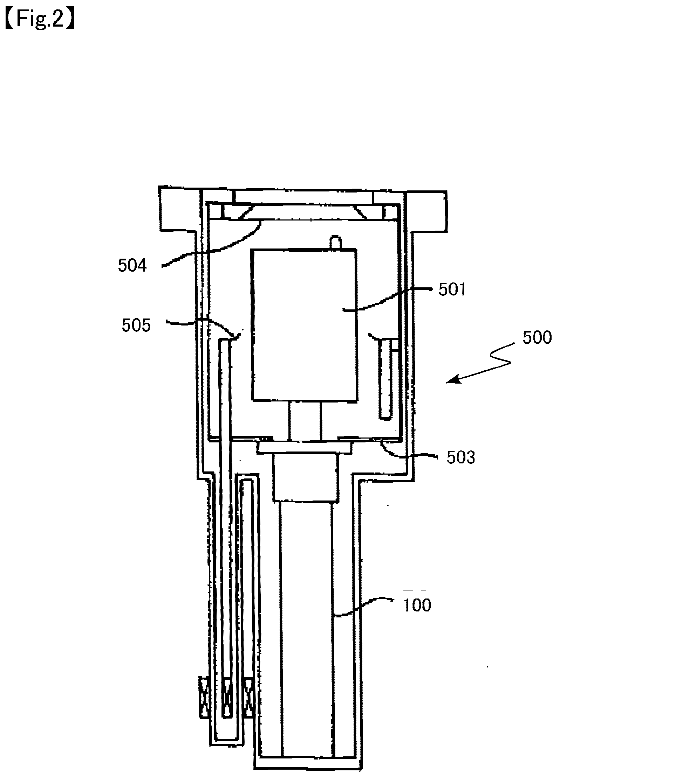

FIG. 2 is a cross-sectional view illustrating a schematic configuration of a cryopump according to a fifth embodiment. The cryopump according to the fifth embodiment is a cryopump 500 including the cold storage cryogenic refrigerator 100 according to the fourth embodiment.

The cryopump 500 includes a cryopanel 501 that condenses or adsorbs gas molecules, a cold storage cryogenic refrigerator 100 that cools the cryopanel 501 to a predetermined cryogenic temperature, a shield 503 provided between the cryopanel 501 and the cold storage cryogenic refrigerator 100, a baffle 504 provided at an intake port, and a ring 505 that changes an exhaust speed of argon, nitrogen, hydrogen, or the like.

According to the fifth embodiment, a cryopump having excellent characteristics can be realized by using a refrigerator having excellent characteristics. In addition, by using the cryopump according to the fifth embodiment in a semiconductor manufacturing device, the long-term reliability of the semiconductor manufacturing device can be improved.

### (Sixth Embodiment)

A superconducting magnet according to a sixth embodiment includes the refrigerator according to the fourth embodiment. Hereinafter, some of the explanations overlapping with those in the fourth embodiment will be omitted.

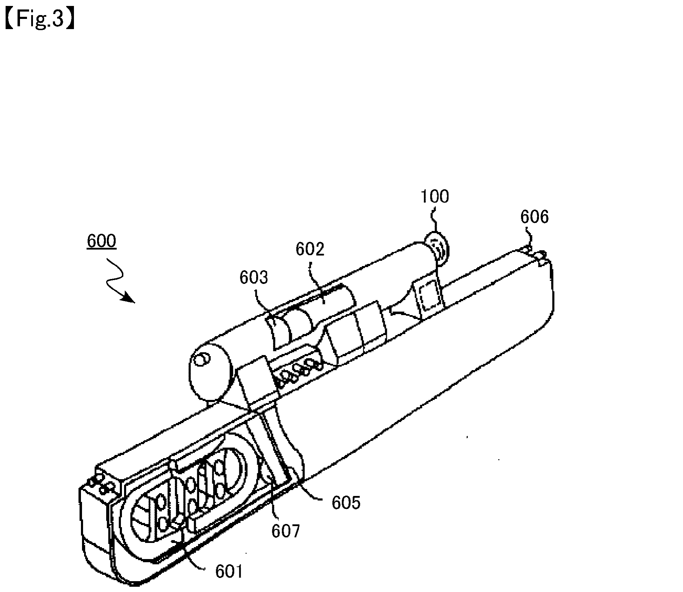

FIG. 3 is a perspective view illustrating a schematic configuration of the superconducting magnet according to the sixth embodiment. The superconducting magnet according to the sixth embodiment is, for example, a superconducting magnet 600 for magnetically levitated train including the cold storage cryogenic refrigerator 100 according to the fourth embodiment.

The superconducting magnet 600 for magnetically levitated train includes a superconducting coil 601, a liquid helium tank 602 for cooling the superconducting coil 601, a liquid nitrogen tank 603 for preventing volatilization of liquid helium, a laminated heat insulating material 605, a power lead 606, a permanent current switch 607, and a cold storage cryogenic refrigerator 100.

According to the sixth embodiment, a superconducting magnet having excellent characteristics can be realized by using a refrigerator having excellent characteristics.

### (Seventh Embodiment)

A nuclear magnetic resonance imaging apparatus according to a seventh embodiment includes the refrigerator according to the fourth embodiment. Hereinafter, some of the explanations overlapping with those in the fourth embodiment will be omitted.

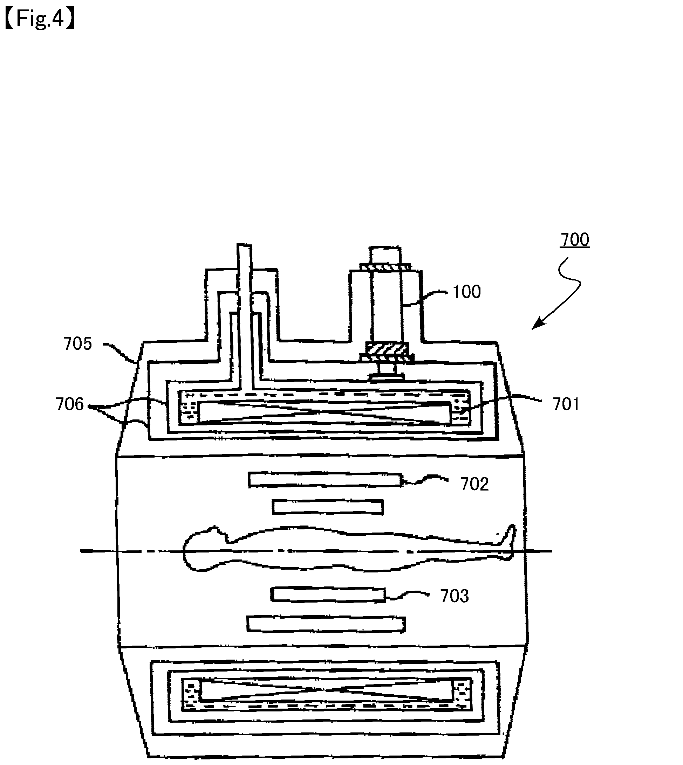

FIG. 4 is a cross-sectional view illustrating a schematic configuration of the nuclear magnetic resonance imaging apparatus according to the seventh embodiment. The nuclear magnetic resonance imaging (MRI) apparatus according to the seventh embodiment is a nuclear magnetic resonance imaging apparatus 700 including the cold storage cryogenic refrigerator 100 according to the fourth embodiment.

The nuclear magnetic resonance imaging apparatus 700 includes a superconducting static magnetic field coil 701 that applies a spatially uniform and temporally stable static magnetic field to a human body, a correction coil (not illustrated) that corrects nonuniformity of the generated magnetic field, a gradient magnetic field coil 702 that gives a magnetic field gradient to a measurement region, a radio wave transmission/reception probe 703, a cryostat 705, and a radiation adiabatic shield 706. In addition, the cold storage cryogenic refrigerator 100 is used for cooling the superconducting static magnetic field coil 701.

According to the seventh embodiment, a nuclear magnetic resonance imaging apparatus having excellent characteristics can be realized by using a refrigerator having excellent characteristics.

### (Eighth Embodiment)

A nuclear magnetic resonance apparatus according to an eighth embodiment includes the refrigerator according to the fourth embodiment. Hereinafter, some of the explanations overlapping with those in the fourth embodiment will be omitted.

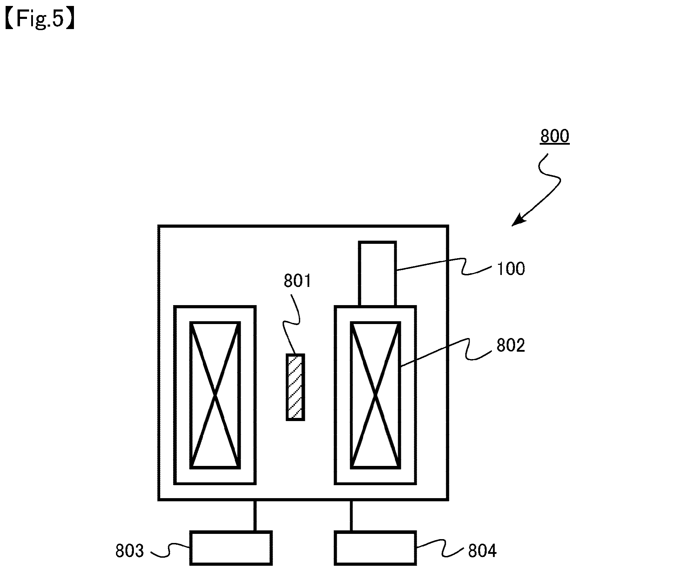

FIG. 5 is a cross-sectional view illustrating a schematic configuration of the nuclear magnetic resonance apparatus according to the eighth embodiment. The nuclear magnetic resonance (NMR) apparatus according to the eighth embodiment is a nuclear magnetic resonance apparatus 800 including the cold storage cryogenic refrigerator 100 according to the fourth embodiment.

The nuclear magnetic resonance apparatus 800 includes a superconducting static magnetic field coil 802 that applies a magnetic field to a sample such as an organic substance placed in a sample tube 801, a high frequency oscillator 803 that applies a radio wave to the sample tube 801 in the magnetic field, and an amplifier 804 that amplifies an induced current generated in a coil (not illustrated) around the sample tube 801. In addition, the nuclear magnetic resonance apparatus 800 includes a cold storage cryogenic refrigerator 100 that cools the superconducting static magnetic field coil 802.

According to the eighth embodiment, a nuclear magnetic resonance apparatus having excellent characteristics can be realized by using a refrigerator having excellent characteristics.

### (Ninth Embodiment)

A magnetic field application type single crystal pulling apparatus according to a ninth embodiment includes the refrigerator according to the fourth embodiment. Hereinafter, some of the explanations overlapping with those in the fourth embodiment will be omitted.

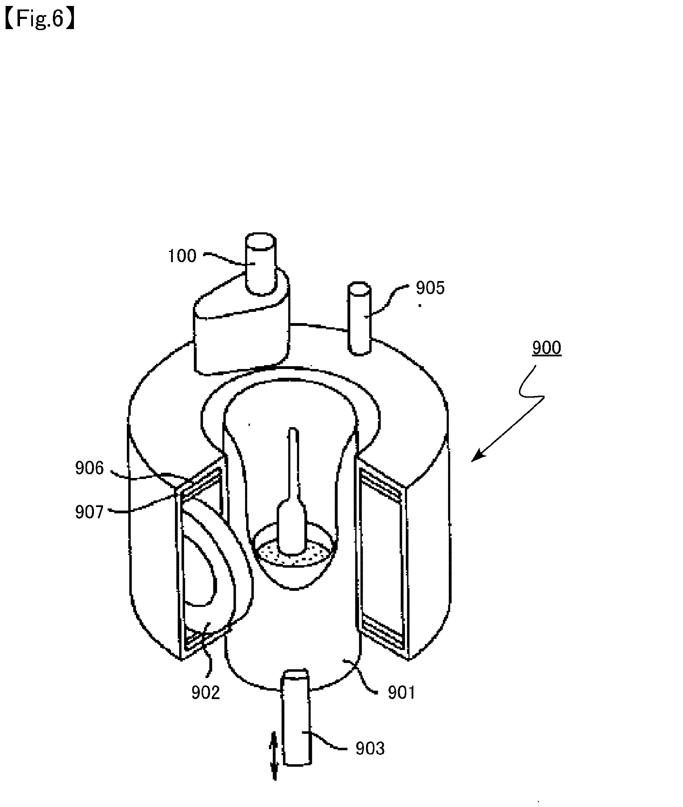

FIG. 6 is a perspective view illustrating a schematic configuration of the magnetic field application type single crystal pulling apparatus according to the ninth embodiment. The magnetic field application type single crystal pulling apparatus according to the ninth embodiment is a magnetic field application type single crystal pulling apparatus 900 including the cold storage cryogenic refrigerator 100 according to the fourth embodiment.

The magnetic field application type single crystal pulling apparatus 900 includes a single crystal pulling unit 901 having a raw material melting crucible, a heater, a single crystal pulling mechanism, etc., a superconducting coil 902 that applies a static magnetic field to a raw material melt, a lifting mechanism 903 for the single crystal pulling unit 901, a current lead 905, a heat shield plate 906, and a helium container 907. In addition, the cold storage cryogenic refrigerator 100 is used for cooling the superconducting coil 902.

According to the ninth embodiment, a magnetic field application type single crystal pulling apparatus having excellent characteristics can be realized by using a refrigerator having excellent characteristics.

### (Tenth Embodiment)

A helium re-condensing device according to a tenth embodiment includes the refrigerator according to the fourth embodiment. Hereinafter, some of the explanations overlapping with those in the fourth embodiment will be omitted.

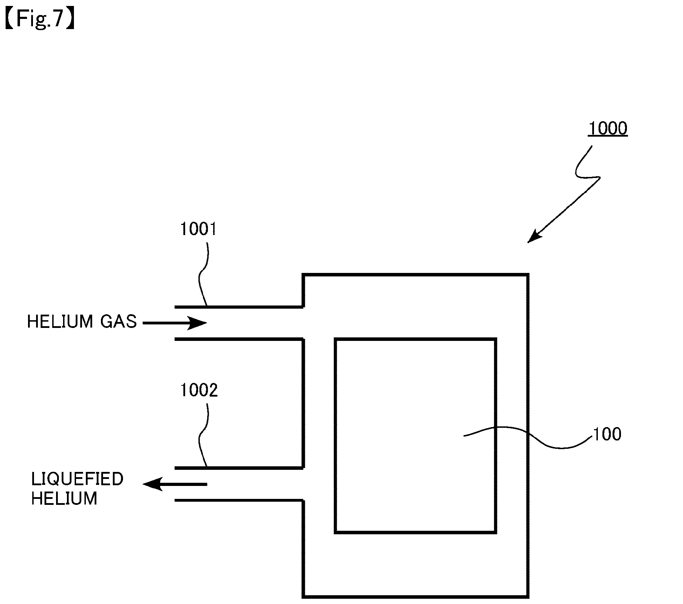

FIG. 7 is a schematic diagram illustrating a schematic configuration of the helium re-condensing device according to the tenth embodiment. The helium re-condensing device according to the tenth embodiment is a helium re-condensing device 1000 including the cold storage cryogenic refrigerator 100 according to the fourth embodiment.

The helium re-condensing device 1000 includes a cold storage cryogenic refrigerator 100, an evaporation pipe 1001, and a liquefaction pipe 1002.

The helium re-condensing device 1000 can recondense helium gas into liquid helium, the helium gas being evaporated from a device using liquid helium, e.g., a superconducting magnet, or a liquid helium device included in a device using a superconducting magnet such as a nuclear magnetic resonance (NMR) device, a nuclear magnetic resonance imaging (MRI) device, a physical property measurement system (PPMS), or a magnetic property measurement system.

The helium gas is introduced from a liquid helium device (not illustrated) into the helium re-condensing device 1000 through the evaporation pipe 1001. The helium gas is cooled to 4 K or less, which is a temperature for liquefaction of helium, by the cold storage cryogenic refrigerator 100. The condensed and liquefied liquid helium returns to the liquid helium device through the liquefaction pipe 1002.

According to the tenth embodiment, a helium re-condensing device having excellent characteristics can be realized by using a refrigerator having excellent characteristics.

### (Eleventh Embodiment)

A dilution refrigerator according to an eleventh embodiment includes the refrigerator according to the fourth embodiment. Hereinafter, some of the explanations overlapping with those in the fourth embodiment will be omitted.

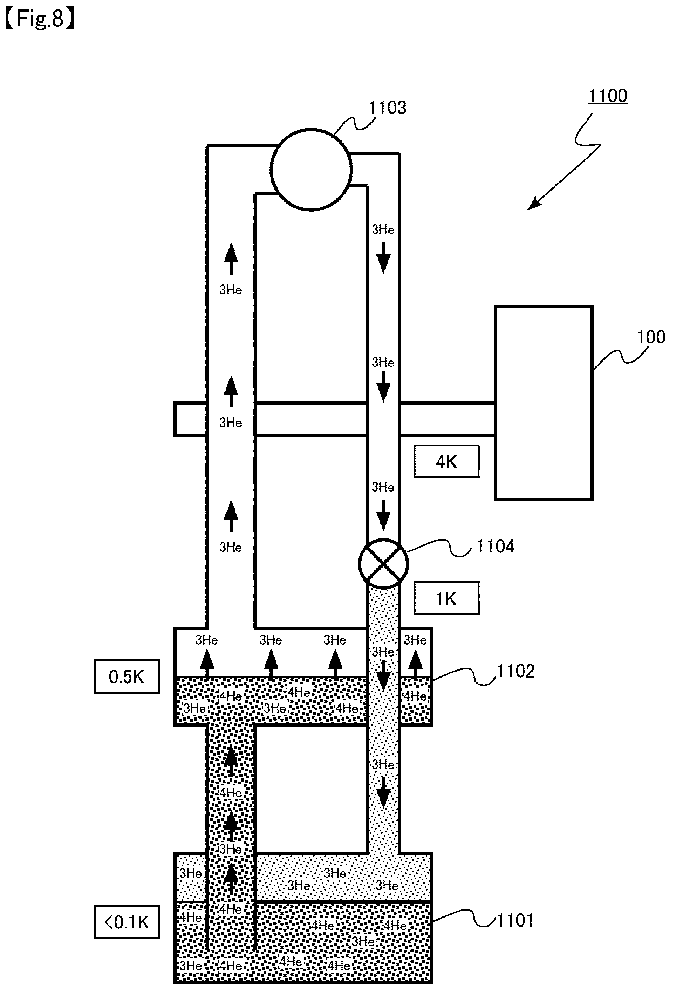

FIG. 8 is a schematic diagram illustrating a schematic configuration of the dilution refrigerator according to the eleventh embodiment. The dilution refrigerator according to the eleventh embodiment is a dilution refrigerator 1100 including the cold storage cryogenic refrigerator 100 according to the fourth embodiment.

The dilution refrigerator 1100 includes a mixing chamber 1101, a distillation chamber 1102, a circulation pump 1103, a Joule-Thomson valve 1104, and a cold storage cryogenic refrigerator 100.

Helium exists in two isotopes: normal helium 4 (4He) having an atomic weight of 4, and light helium 3 (3He) having an atomic weight of 3. The dilution refrigerator 1100 can realize a cryogenic temperature of, for example, less than 0.1 K by using dilution heat generated when mixing helium 4 and helium 3.

A mixed liquid of liquid helium 4 and liquid helium 3 exists in the mixing chamber 1101. In the mixing chamber 1101, an interface exists between liquid helium 4 and liquid helium 3 that are phase-separated. The mixing chamber 1101 has the lowest temperature. The temperature of the mixing chamber is, for example, less than 0.1 K.

The distillation chamber 1102 is connected to the mixing chamber 1101. The distillation chamber 1102 is maintained at, for example, 0.5 K. In the distillation chamber 1102, only helium 3 selectively evaporates and becomes gas.

The circulation pump 1103 has a function of circulating helium 3 that has become gas.

The cold storage cryogenic refrigerator 100 has a function of cooling helium 3 that has become gas to, for example, 4 K.

The Joule-Thomson valve 1104 has a function of liquefying helium 3 that has been cooled to, for example, 4 K.

The dilution refrigerator 1100 can realize a cryogenic temperature of, for example, less than 0.1 K, by forcibly dissolving liquid helium 3 in liquid helium 4 in the mixing chamber 1101.

According to the eleventh embodiment, a dilution refrigerator having excellent characteristics can be realized by using a refrigerator having excellent characteristics.

### Examples

Hereinafter, examples of the cold storage material according to the first embodiment or the cold storage material particle according to the second embodiment, comparative examples, and results of evaluation thereof will be described.

### (Example 1)

A Gd₂O₃ powder, an Al₂O₃ powder, and a Na₂CO₃ powder were mixed and pulverized in a ball mill for 24 hours to prepare a raw material mixture. Next, the obtained raw material mixture was dried and then granulated using a rolling granulator to prepare granulated particles having a particle diameter of 0.3 mm to 0.5 mm. At this time, polyvinyl alcohol was used as a binder, and added in an amount of 1.2 wt% to the raw material powders. The granulated particles had a sodium concentration of 0.52 atom% and a carbon concentration of 0.99 wt%. The obtained raw material mixture was molded to obtain a molded body.

In order to evaluate the strength of the granulated particles, the granulated particles were filled in a cylindrical container having a diameter of 15 mm and a height of 5 mm. At this time, the cold storage material was filled in a sufficient amount so that the granulated particles were fixed in the cylindrical container to prevent the granulated particles from moving freely. A single vibration having an amplitude of 2 mm and a maximum acceleration of 200m/s² was applied to the container 1×10³ times. As a result, the proportion of the broken cold storage material was less than 0.1 wt%.

The granulated particles and the molded body were degreased at 600°C for 6 hours in an air atmosphere. The granulated particles after being degreased and the molded body had a sodium concentration of 0.54 atom% and a carbon concentration of 0.51 wt%. Heat treatment was performed at 1300°C for 12 hours in a pressurized inert gas atmosphere to sinter the particles and the molded body.

The main component of the cold storage material and the cold storage material particles in Example 1 was a gadolinium aluminum oxide. The sodium concentration of the cold storage material and the cold storage material particles in Example 1 was 0.55 atom%.

A maximum volumetric specific heat value at 10 K or less and a thermal conductivity at 4.2 K of the cold storage material according to Example 1 were measured. The measurement was performed using a physical property measurement system (PPMS).

A refrigerator according to Example 1 was assembled by filling cold storage material particles according to Example 1 in an amount of 250 g on the low-temperature side of the second-stage cold storage device of the two-stage GM refrigerator illustrated in FIG. 1, while filling a Pb cold storage material in an amount of 250 g on the high-temperature side of the second-stage cold storage device, and a refrigeration test was performed to measure a refrigeration capacity at 4.2 K. Note that a thermal load was applied to the first-stage cold storage device so that the temperature was 50 K.

As a result of the refrigeration test, 0.66 W was obtained as a refrigeration capacity at 4.2 K.

In the following examples and comparative examples, a time for which the raw material powders are mixed, a condition for heat treatment for sintering, etc. are adjusted to be appropriate conditions. In addition, the conditions for testing the refrigerator were set to be the same.

### (Example 2)

A cold storage material and cold storage material particles were produced in the same manner as in Example 1, except that a Li₂CO₃ powder was used instead of the Na₂CO₃ powder.

### (Example 3)

A cold storage material and cold storage material particles were produced in the same manner as in Example 1, except that a K₂CO₃ powder was used instead of the Na₂CO₃ powder.

### (Example 4)

A cold storage material and cold storage material particles were produced in the same manner as in Example 1, except that a CaCO₃ powder was used in addition to the Na₂CO₃ powder.

### (Example 5)

A cold storage material and cold storage material particles were produced in the same manner as in Example 4, except that a Li₂CO₃ powder was used instead of the Na₂CO₃ powder.

### (Example 6)

A cold storage material and cold storage material particles were produced in the same manner as in Example 4, except that a K₂CO₃ powder was used instead of the Na₂CO₃ powder.

### (Example 7)

A cold storage material and cold storage material particles were produced in the same manner as in Example 4, except that a MgCO₃ powder was used instead of the CaCO₃ powder.

### (Example 8)

A cold storage material and cold storage material particles were produced in the same manner as in Example 4, except that a SrCO₃ powder was used instead of the CaCO₃ powder.

### (Example 9)

A cold storage material and cold storage material particles were produced in the same manner as in Example 4, except that a BaCO₃ powder was used instead of the CaCO₃ powder.

### (Example 10)

A cold storage material and cold storage material particles were produced in the same manner as in Example 1, except that a Tb₂O₃ powder was used instead of the Gd₂O₃ powder.

### (Example 11)

A cold storage material and cold storage material particles were produced in the same manner as in Example 1, except that a Dy₂O₃ powder was used instead of the Gd₂O₃ powder.

### (Example 12)

A cold storage material and cold storage material particles were produced in the same manner as in Example 1, except that a Ho₂O₃ powder was used instead of the Gd₂O₃ powder.

### (Example 13)

A cold storage material and cold storage material particles were produced in the same manner as in Example 1, except that a K₂CO₃ powder was used in addition to the Na₂CO₃ powder.

### (Example 14)

A cold storage material and cold storage material particles were produced in the same manner as in Example 1, except that a Li₂CO₃ powder was used in addition to the Na₂CO₃ powder.

### (Example 15)

A cold storage material and cold storage material particles were produced in the same manner as in Example 1, except that a K₂CO₃ powder and a CaCO₃ powder were used in addition to the Na₂CO₃ powder.

### (Example 16)

A cold storage material and cold storage material particles were produced in the same manner as in Example 1, except that a CaCO₃ powder and a SrCO₃ powder were used in addition to the Na₂CO₃ powder.

### (Examples 17 to 19)

A cold storage material and cold storage material particles were produced in the same manner as in Example 1, except that the weight of the Na₂CO₃ powder was reduced.

### (Examples 20 to 22)

A cold storage material and cold storage material particles were produced in the same manner as in Example 1, except that the weight of the Na₂CO₃ powder was increased.

### (Examples 23 and 24)

A cold storage material and cold storage material particles were produced in the same manner as in Example 4, except that the weight of the CaCO₃ powder was reduced.

### (Examples 25 to 27)

A cold storage material and cold storage material particles were produced in the same manner as in Example 4, except that the weight of the CaCO₃ powder was increased.

### (Examples 28 to 30)

A cold storage material and cold storage material particles were produced in the same manner as in Example 1, except that some of the Gd₂O₃ powder was changed to Tb₂O₃, Dy₂O₃, or Ho₂O₃.

### (Example 31)

A slurry was prepared by adding a Gd₂O₃ powder and an Al₂O₃ powder to a sodium alginate aqueous solution and mixing them for 12 hours. The sodium alginate aqueous solution was added so that the amount of sodium alginate was 2.3 wt% with respect to the raw material powders. The prepared slurry was dropped into a calcium lactate aqueous solution as a gelling solution. A syringe was used for dropping the slurry. The diameter of the syringe was 510 µm, and the distance from the tip of the syringe to the liquid level of the calcium lactate aqueous solution was 100 mm. In addition, the slurry was filled in a mold and then immersed in the gelling solution.

The slurry dropped by the syringe and the slurry filled in the mold were kept in the gelling solution for 5 hours.

Thereafter, the gelled granulated particles were washed with pure water. The slurry filled in the mold was removed from the mold and then washed with pure, thereby obtaining a molded body. After washing the molded body and the particles, the molded body and the particles were dried. The granulated particles had a sodium concentration of 0.78 atom% and a carbon concentration of 0.82 wt%. After the molded body and the particles were dried, the molded body and the particles were degreased and sintered.

The degreasing was performed at 600°C for 6 hours in an air atmosphere, and after the degreasing, the granulated particles had a sodium concentration of 1.0 atom% and a carbon concentration of 0.54 wt%. After the degreasing, heat treatment was performed at 1300°C for 12 hours in a pressurized inert gas atmosphere to sinter the molded body and the particles. The main component of the cold storage material and the cold storage material particles in Example 32 is a gadolinium aluminum oxide. The sodium concentration of the cold storage material and the cold storage material particles in Example 32 was 0.83 atom%.

### (Example 32)

A cold storage material and cold storage material particles were produced in the same manner as in Example 31, except that a potassium alginate aqueous solution was used instead of the sodium alginate aqueous solution.

### (Examples 33 to 37)

A cold storage material and cold storage material particles were produced in the same manner as in Example 32, except that the amount of the potassium alginate aqueous solution was increased or reduced.

### (Example 38)

A cold storage material and cold storage material particles were produced in the same manner as in Example 31, except that a magnesium chloride aqueous solution was used instead of the calcium lactate aqueous solution.

### (Example 39)

A cold storage material and cold storage material particles were produced in the same manner as in Example 31, except that a strontium chloride aqueous solution was used instead of the calcium lactate aqueous solution.

### (Example 40)

A cold storage material and cold storage material particles were produced in the same manner as in Example 31, except that a barium chloride aqueous solution was used instead of the calcium lactate aqueous solution.

### (Example 41)

A cold storage material and cold storage material particles were produced in the same manner as in Example 31, except that a K₂CO₃ powder was added as a raw material powder.

### (Example 42)

A cold storage material and cold storage material particles were produced in the same manner as in Example 31, except that a Li₂CO₃ powder was added as a raw material powder.

### (Example 43)

A cold storage material and cold storage material particles were produced in the same manner as in Example 31, except that an air pulse type dispenser was used rather than the syringe as a method of filling a slurry in a mold and a method of dropping a slurry. The diameter of the nozzle was 510 µm, and the distance from the tip of the nozzle to the liquid level of the calcium lactate aqueous solution was 100 mm.

### (Example 44)

A cold storage material and cold storage material particles were produced in the same manner as in Example 31, except that a piezo type dispenser was used rather than the syringe as a method of filling a slurry in a mold and a method of dropping a slurry. The diameter of the nozzle was 510 µm, and the distance from the tip of the nozzle to the liquid level of the calcium lactate aqueous solution was 100 mm.

### (Examples 45 to 50)

Cold storage material particles in Examples 45 to 50 have different particle diameters or different aspect ratio from the cold storage material particles in Example 43. When the cold storage material particles in Examples 45 to 50 were produced, the diameter of the syringe and the distance from the tip of the syringe to the surface of the gelling solution were changed as compared with those when the cold storage material particles were produced in Example 43.

### (Example 51)

A cold storage material and cold storage material particles were produced in the same manner as in Example 31, except that a continuous type inkjet was used rather than the syringe as a method of filling a slurry in a mold and a method of dropping a slurry. The diameter of the nozzle was 510 µm, and the distance from the tip of the nozzle to the liquid level of the calcium lactate aqueous solution was 100 mm.

### (Examples 52 to 55)

A cold storage material and cold storage material particles were produced in the same manner as in Example 1, except that the weight of polyvinyl alcohol was changed. In addition, a strength of a granulated particle was measured in the same manner as in Example 1.

### (Examples 56 to 59)

A cold storage material and cold storage material particles were produced in the same manner as in Example 32, except that the amount of sodium alginate with respect to the raw material powders was changed by changing the concentration of the sodium alginate aqueous solution or the ratio of the sodium alginate aqueous solution with respect to the raw material powders. In addition, a strength of a granulated particle was measured in the same manner as in Example 1.

### (Example 60)

A cold storage material and cold storage material particles were produced in the same manner as in Example 1, except that a ZrO₂ powder was also used as a raw material powder. The ZrO₂ powder was added so that Zr was contained in an amount of 15 atom% in the cold storage material particles.

### (Example 61)

A cold storage material and cold storage material particles were produced in the same manner as in Example 4, except that a ZrO₂ powder was also used as a raw material powder. The ZrO₂ powder was added so that Zr was contained in an amount of 15 atom% in the cold storage material particles.

### (Example 62)

A cold storage material and cold storage material particles were produced in the same manner as in Example 31, except that a ZrO₂ powder was also added to the sodium alginate aqueous solution. The ZrO₂ powder was added so that Zr was contained in an amount of 0.01 atom% in the cold storage material particles.

### (Comparative Example 1)

A cold storage material and cold storage material particles in Comparative Example 1 are different from the cold storage material and the cold storage material particles in Example 1 in that the atomic concentration of sodium is as low as 0.0008 atom%. When the cold storage material and the cold storage material particles were produced in Comparative Example 1, the weight of the Na₂CO₃ powder was reduced as compared with that when the cold storage material and the cold storage material particles were produced in Example 1.

### (Comparative Example 2)

A cold storage material and cold storage material particles in Comparative Example 2 are different from the cold storage material and the cold storage material particles in Example 1 in that the atomic concentration of sodium is as high as 15 atom%. When the cold storage material and the cold storage material particles were produced in Comparative Example 2, the weight of the Na₂CO₃ powder was increased as compared with that when the cold storage material and the cold storage material particles were produced in Example 1. At this time, in addition to GdAlO₃, was remarkably generated.

### (Comparative Example 3)

A cold storage material and cold storage material particles in Comparative Example 3 are different from the cold storage material and the cold storage material particles in Example 4 in that the atomic concentration of calcium is as high as 15 atom%. When the cold storage material and the cold storage material particles were produced in Comparative Example 3, the weight of the CaCO₃ powder was increased as compared with that when the cold storage material and the cold storage material particles were produced in Example 4.

### (Comparative Example 4)

A cold storage material and cold storage material particles in Comparative Example 4 are different from the cold storage material and the cold storage material particles in Example 1 in that they do not contain a Group 1 element. The cold storage material and the cold storage material particles in Comparative Example 4 were produced without using a powder containing a Group 1 element.

### (Comparative Example 5)

The cold storage material granulated particles in Comparative Example 5 are different from those in Example 1 in that the amount of carbon is as large as 25 wt%. The granulated particles in Comparative Example 5 had a carbon concentration of 12 wt% after being degreased, but the particles were cracked or chipped after being sintered, and the particles could not be recovered. For this reason, evaluation of specific heat and strength and refrigerator testing could not be carried out. When the cold storage material and the cold storage material particles were produced in Comparative Example 5, the weight ratio of polyvinyl alcohol used as a binder was increased to 34 wt% with respect to the raw material powders.

### (Comparative Example 6)

A cold storage material granulated body in Comparative Example 6 is different from that in Example 1 in that the amount of carbon is as small as 0.005 wt%. In Comparative Example 6, particles could not be recovered after being granulated. For this reason, evaluation of specific heat and strength and refrigerator testing could not be carried out. When the cold storage material and the cold storage material particles were produced in Comparative Example 6, the weight ratio of polyvinyl alcohol with respect to the raw material powders was 0.2 wt%.

### (Comparative Example 7)

Cold storage material granulated particles after being subjected to degreasing treatment in Comparative Example 7 are different from those in Example 1 in that the amount of carbon is as large as 15 wt%. The carbon concentration before the degreasing was 19 wt%. The granulated particles in Comparative Example 7 were sintered, thereby obtaining a cold storage material and cold storage material particles. When the cold storage material and the cold storage material particles were produced in Comparative Example 7, degreasing was performed at 400°C for 1 hour in an air atmosphere with polyvinyl alcohol in an amount of 24 wt% with respect to the raw material powders.

### (Comparative Example 8)

Cold storage material particle after being degreased in Comparative Example 8 are different from those in Example 1 in that the amount of carbon is small as below the detection limit. The granulated particles in Comparative Example 8 were cracked or chipped after being degreased. The carbon concentration before the degreasing was 0.01 wt%. When the cold storage material and the cold storage material particles were produced in Comparative Example 8, degreasing was performed at 800°C for 12 hours in an air atmosphere with polyvinyl alcohol in an amount of 0.2 wt% with respect to the raw material powders.

### (Comparative Example 9)

A cold storage material and cold storage material particles in Comparative Example 9 are different from those in Example 60 in that the amount of Zr contained in the cold storage material particles is as large as 25 atom%. When the cold storage material and the cold storage material particles were produced in Comparative Example 9, the weight of the ZrO₂ powder was increased as compared with that when the cold storage material and the cold storage material particles were produced in Example 60.

Maximum volumetric specific heat values at 10 K or less and thermal conductivities at 4.2 K of the cold storage materials according to the examples and the comparative examples were measured. The results are shown in Table 1, Table 2, and Table 3.

Refrigeration capacities of the cold storage material particles according to the examples and the comparative examples are shown in Table 1, Table 2, Table 3, and Table 4. A refrigerator was assembled by filling cold storage material particles according to each of the examples and the comparative examples in an amount of 250 g on the low-temperature side of the second-stage cold storage device of the two-stage GM refrigerator illustrated in FIG. 1, while filling a Pb cold storage material in an amount of 250 g on the high-temperature side of the second-stage cold storage device, and a refrigeration test was performed to measure a refrigeration capacity at 4.2 K. Note that a thermal load was applied to the first-stage cold storage device so that the temperature was 50 K.

The results of evaluating strengths of the cold storage material granulated particles before being degreased and after being degreased according to the examples and the comparative examples are shown in Table 3. The granulated particles according to each of the examples and the comparative examples were filled in a cylindrical container having a diameter of 15 mm and a height of 5 mm. At this time, the cold storage material was filled in a sufficient amount so that the granulated particles were fixed in the cylindrical container to prevent the granulated particles from moving freely. A single vibration having an amplitude of 2 mm and a maximum acceleration of 200m/s² was applied to the container 1×10³ times. As a result, the ratio of broken cold storage material granulated particles was evaluated.

**[Table 1]**

| | Granulation method | Phase of cold storage material | Type of Group 1 element | Concentration of Group 1 element in particle (atm %) | Type of Group 2 element | Concentration of Group 2 element in particle (atom %) | Particle diameter of cold storage material particle (µm) | Aspect ratio of cold storage material particle | Maximum volumetric specific heat value at 10 K or less (J/cm³. K) | Thermal conductivity at 4.2 K (W/cm.K) | Refrigeration capacity at 4.2 K (W) |
|---|---|---|---|---|---|---|---|---|---|---|---|
| Example 1 | Rolling granulation | GdAlO3 | Na | 0.55% | | | 250 | 1 | 1.2 | 0.01 | 0.66 |
| Example 2 | Rolling granulation | GdAlO3 | Li | 0.45% | | | 400 | 1 | 1.2 | 0.011 | 0.66 |
| Example 3 | Rolling granulation | GdAlO3 | K | 0.87% | | | 260 | 1 | 1.2 | 0.012 | 0.66 |
| Example 4 | Rolling granulation | GdAlO3 | Na | 0.59% | Ca | 0.004% | 440 | 1.1 | 1.2 | 0.015 | 0.69 |
| Example 5 | Rolling granulation | GdAlO3 | Li | 0.47% | Ca | 0.003% | 220 | 1.2 | 1.2 | 0.014 | 0.68 |
| Example 6 | Rolling granulation | GdAlO3 | K | 0.83% | Ca | 0.005% | 242 | 1.1 | 1.2 | 0.015 | 0.69 |
| Example 7 | Rolling granulation | GdAlO3 | Na | 0.43% | Mg | 0.010% | 360 | 1 | 1.2 | 0.016 | 0.69 |
| Example 8 | Rolling granulation | GdAlO3 | Na | 0.41% | Sr | 0.012% | 460 | 1.5 | 1.2 | 0.016 | 0.69 |
| Example 9 | Rolling granulation | GdAlO3 | Na | 0.47% | Ba | 0.060% | 300 | 1.3 | 1.2 | 0.018 | 0.69 |
| Example 10 | Rolling granulation | TbAlO3 | Na | 0.52% | | | 1000 | 1.6 | 1.75 | 0.01 | 0.62 |
| Example 11 | Rolling granulation | DyAlO3 | Na | 0.51% | | | 50 | 1.1 | 1 | 0.01 | 0.61 |
| Example 12 | Rolling granulation | HoAlO3 | Na | 0.55% | | | 450 | 1 | 1.25 | 0.01 | 0.59 |
| Example 13 | Rolling granulation | GdAlO3 | Na | 0.76% | | | 352 | 1 | 1.2 | 0.014 | 0.67 |
| | | | K | 0.82% | | | | | | | |
| Example 14 | Rolling granulation | GdAlO3 | Na | 0.55% | | | 280 | 1.4 | 1.2 | 0.014 | 0.67 |
| | | | Li | 0.61% | | | | | | | |
| Example 15 | Rolling granulation | GdAlO3 | Na | 0.62% | Ca | 0.16% | 421 | 1.2 | 1.2 | 0.18 | 0.71 |
| | | | K | 0.64% | | | | | | | |
| Example 16 | Rolling granulation | GdAlO3 | Na | 0.71% | Ca | 0.15% | 392 | 1.1 | 1.2 | 0.19 | 0.71 |
| | | | | | Sr | 0.12% | | | | | |
| Example 17 | Rolling granulation | GdAlO3 | Na | 0.10% | | | 251 | 1 | 1.2 | 0.009 | 0.66 |
| Example 18 | Rolling granulation | GdAlO3 | Na | 0.01% | | | 250 | 1 | 1.2 | 0.009 | 0.66 |
| Example 19 | Rolling granulation | GdAlO3 | Na | 0.002% | | | 240 | 1 | 1.2 | 0.009 | 0.66 |
| Example 20 | Rolling granulation | GdAlO3 | Na | 1.00% | | | 260 | 1 | 1 | 0.015 | 0.69 |
| Example 21 | Rolling granulation | GdAlO3 | Na | 5.00% | | | 250 | 1 | 0.95 | 0.015 | 0.69 |
| Example 22 | Rolling granulation | GdAlO3 | Na | 9.80% | | | 243 | 1 | 0.9 | 0.015 | 0.69 |
| Example 23 | Rolling granulation | GdAlO3 | Na | 0.59% | Ca | 0.003% | 440 | 1.1 | 1.2 | 0.015 | 0.69 |
| Example 24 | Rolling granulation | GdAlO3 | Na | 0.59% | Ca | 0.001% | 430 | 1.1 | 1.2 | 0.015 | 0.69 |
| Example 25 | Rolling granulation | GdAlO3 | Na | 0.59% | Ca | 1.00% | 420 | 1.1 | 1 | 0.019 | 0.71 |
| Example 26 | Rolling granulation | GdAlO3 | Na | 0.59% | Ca | 4.90% | 450 | 1.1 | 0.95 | 0.02 | 0.72 |
| Example 27 | Rolling granulation | GdAlO3 | Na | 0.59% | Ca | 9.90% | 440 | 1.1 | 0.9 | 0.021 | 0.73 |
| Example 28 | Rolling granulation | (Gd, Tb)AlO3 | Na | 0.55% | | | | 1 | 1.3 | 0.01 | 0.66 |
| Example 29 | Rolling granulation | (Gd, Dy)AlO3 | Na | 0.55% | | | | 1 | 1.1 | 0.01 | 0.66 |
| Example 30 | Rolling granulation | (Gd, Ho)AlO3 | Na | 0.55% | | | | 1 | 1.1 | 0.01 | 0.66 |

**[Table 2]**

| | Granulation method | Phase of cold storage material | Type of Group 1 element | Concentration of Group 1 element in particle | Type of Group 2 element | Concentration of Group 2 element in particle | Particle size of cold storage material particle | Aspect ratio of cold storage material particle | Maximum volumetric specific heat value at 10 K or less | Thermal conductivity at 4.2 K | Refrigeration capacity at 4.2 K |
|---|---|---|---|---|---|---|---|---|---|---|---|
| | | | | (atom % ) | | (atom %) | (µm) | | (J/cm³. K) | (Wicm. K) | (W) |
| Example 31 | Syringe dropping | GdAlO3 | Na | 0.83% | Ca | 0.16% | 330 | 1.1 | 1.2 | 0.013 | 0.66 |
| Ex ample 32 | Syringe dropping | GdAlO3 | K | 0.10% | Ca | 0.17% | 280 | 1.2 | 1.2 | 0.014 | 0.67 |
| Ex ample 33 | Syringe dropping | GdAlO3 | K | 0.01% | Ca | 0.17% | 280 | 1.2 | 1.2 | 0.01 | 0.66 |
| Ex ample 34 | Syringe dropping | GdAlO3 | K | 0.002% | Ca | 0.17% | 280 | 1.2 | 1.2 | 0.009 | 0.66 |
| Ex ample 35 | Syringe dropping | GdAlO3 | K | 2.00% | Ca | 0.17% | 280 | 1.2 | 1.1 | 0.015 | 0.69 |
| Ex ample 36 | Syringe dropping | GdAlO3 | K | 5.00% | Ca | 0.17% | 280 | 1.2 | 0.8 | 0.019 | 0.71 |
| Ex ample 37 | Syringe dropping | GdAlO3 | K | 9.90% | Ca | 0.17% | 280 | 1.2 | 0.55 | 0.02 | 0.72 |
| Ex ample 38 | Syringe dropping | GdAlO3 | Na | 0.79% | Mg | 0.21% | 440 | 1 | 1.2 | 0.012 | 0.66 |
| Ex ample 39 | Syringe dropping | GdAlO3 | Na | 0.95% | Sr | 0.91% | 390 | 1.3 | 1.2 | 0.014 | 0.67 |
| Example 40 | Syringe dropping | GdAlO3 | Na | 0.75% | Ba | 0.45% | 380 | 1.2 | 1.2 | 0.014 | 0.67 |
| Example 41 | Syringe dropping | GdAlO3 | Na | 0.77% | Ca | 0.16% | 290 | 1.4 | 1.2 | 0.014 | 0.67 |
| | | | K | 0.81% | | | | | | | |
| Ex ample 42 | Syringe dropping | GdAlO3 | Na | 0.89% | Ca | 0.16% | 452 | 1.1 | 1.2 | 0.014 | 0.67 |
| | | | Li | 1.10% | | | | | | | |
| Example 43 | Air pulse type dispenser | GdAlO3 | Na | 0.95% | Ca | 0.16% | 352 | 1 | 1.2 | 0.014 | 0.67 |
| Example 44 | Piezo type dispenser | GdAlO3 | Na | 0.92% | Ca | 0.18% | 256 | 1.1 | 1.2 | 0.014 | 0.67 |
| Example 45 | Air pulse type dispenser | GdAlO3 | Na | 0.95% | Ca | 0.16% | 50 | 1 | 1.2 | 0.014 | 0.66 |
| Example 46 | Air pulse type dispenser | GdAlO3 | Na | 0.95% | Ca | 0.16% | 3000 | 1 | 1.2 | 0.014 | 0.66 |
| Ex ample 47 | Air pulse type dispenser | GdAlO3 | Na | 0.95% | Ca | 0.16% | 40 | 1 | 1.2 | 0.014 | 0.3 |
| Example 48 | Air pulse type dispenser | GdAlO3 | Na | 0.95% | Ca | 0.16% | 4000 | 1 | 1.2 | 0.014 | 0.3 |
| Example 49 | Air pulse type dispenser | GdAlO3 | Na | 0.95% | Ca | 0.16% | 250 | 4.8 | 1.2 | 0.014 | 0.66 |
| Ex ample 50 | Air pulse type dispenser | GdAlO3 | Na | 0.95% | Ca | 0.16% | 250 | 5.5 | 1.2 | 0.014 | 0.3 |
| Example 51 | Continuous type inkjet | GdAlO3 | Na | 0.95% | Ca | 0.16% | 300 | 1 | 1.2 | 0.014 | 0.66 |
| Comparative Example 1 | Rolling granulation | GdAlO3 | Na | 0.0008% | | | 250 | 1 | 1.2 | 0.005 | 0.4 |
| Comparative Example 2 | Rolling granulation | GdAlO3 | Na | 15.00% | | | 250 | 1 | 0.4 | 0.01 | 0.3 |
| Comparative Example 3 | Rolling granulation | GdAlO3 | Na | 0.59% | Ca | 15.00% | 440 | 1.1 | 0.4 | 0.02 | 0.3 |
| Comparative Example 4 | Rolling granulation | GdAlO3 | - | - | - | - | 440 | 1.1 | 1.2 | 0.004 | 0.4 |

**[Table 3]**

| | Granulation method | Phase of cdd storage matrial | Type of Group 1 dement | Concentraton of Group 1 dement in paricle | Type of Group 2 dement | Concentration of Group 2 element in particle (atom %) | Concentration of carbon in particle before degreased (wt%) | Concentration of carbon in particle after degreased (wt%) | Percentage of broken paricles in result of test for strength of granulated paricles before degreased | Percentage of broken paricles in result of test for strength of granulated particles afer degreased | Particle size of cold storage meterial particle | Aspect ratio of cold storage material paricle | Maximum volumetric spaciic heat value at 10 K or less | Thermal conductivity at 4.2 K (W/cm.K) | Refrigeration capacity at 4.2 K (W) |
|---|---|---|---|---|---|---|---|---|---|---|---|---|---|---|---|
| | | | | (atom %) | | | | | (wt%) | wt%) | (µm) | | (J/cm³. K) | | |
| Example 1 | Rolling granulation | GdAlO3 | Na | 0.55% | | | 0.99 wt% | 0.51 wt% | 0.11 wt% | 0.23 wt% | 250 | 1 | 1.2 | 0.01 | 0.66 |
| Example 52 | Rolling granulation | GdAlO3 | Na | 0.55% | | | 19 wt% | 9.5wt% | less than 0.1 wt% | less than 0.1 wt% | 250 | 1 | 1.15 | 0.008 | 0.64 |
| Example 53 | Rolling granulation | GdAlO3 | Na | 0.55% | | | 10 wt% | 4.5wt% | less than 0.1 wt% | less than 0.1 wt% | 250 | 1 | 1.18 | 0.009 | 0.65 |
| Example 54 | Rolling granulation | GdAlO3 | Na | 0.55% | | | 5 wt% | 1 wt% | less than 0.1 wt% | less than 0.1 wt% | 250 | 1 | 1.2 | 0.01 | 0.66 |
| Example 55 | Rolling granulation | GdAlO3 | Na | 0.55% | | | 0.01 wt% | 0.002 wt% | 0.11 wt% | 1 wt% | 250 | 1 | 1.2 | 0.01 | 0.66 |
| Example 31 | Syringe dropping | GdAlO3 | Na | 0.83% | Ca | 0.16% | 0.82 wt% | 0.54 wt% | 0.35 wt% | 0.5 wt% | 330 | 1.1 | 1.2 | 0.013 | 0.66 |
| Example 56 | Syringe dropping | GdAlO3 | Na | 0.83% | Ca | 0.16% | 15 wt% | 9.5wt% | less than 0.1 wt% | less than 0.1 wt% | 330 | 1.1 | 1.15 | 0.008 | 0.64 |
| Example 57 | Syringe dropping | GdAlO3 | Na | 0.83% | Ca | 0.16% | 10 wt% | 4.5wt% | less than 0.1 wt% | less than 0.1 wt% | 330 | 1.1 | 1.18 | 0.009 | 0.65 |
| Example 58 | Syringe dropping | GdAlO3 | Na | 0.83% | Ca | 0.16% | 5 wt% | 1 wt% | less than 0.1 wt% | less than 0.1 wt% | 330 | 1.1 | 1.2 | 0.013 | 0.66 |
| Example 59 | Syringe dropping | GdAlO3 | Na | 0.83% | Ca | 0.16% | 0.01 wt% | 0.003 wt% | 0.11 wt% | 1 wt% | 330 | 1.1 | 1.2 | 0.013 | 0.66 |
| Comparative Example 5 | Rolling granulation | GdAlO3 | Na | 0.55% | | | 25 wt% | 12 wt% | less than 0.1 wt% | less than 0.1 wt% | Not measurable | Not measurable | Not measurable | Not measurable | Not measurable |
| Comparative Example 6 | Rolling granulaion | GdAlO3 | Na | 0.55% | | | 0.005 wt% | | 99 wt% | | Not measurable | Not measurable | Not measurable | Not measurable | Not measurable |
| Comparative Example 7 | Rolling granulation | GdAlO3 | Na | 0.55% | | | 19 wt% | 15 wt% | less than 0.1 wt% | less than 0.1 wt% | 400 | 1.2 | 0.7 | 0.005 | 0.35 |
| Comparative Example 8 | Rolling granulation | GdAlO3 | Na | 0.55% | | | 0.01 wt% | Below detecton limit | 0.11 wt% | 99 wt% | Not measurable | Not measurable | Not measurable | Not measurable | Not measurable |

**[Table 4]**

| | Granulation method | Phase of cold storage material | Type of Group 1 element | Concentration of Group 1 element in particle | Type of Group 2 element | Concentration of Group 2 element in particle | Type of metal or metalloid element derived from sintering aid | Concentration of metal or metalloid element derived from sintering aid | Particle size of cold storage material particle | Aspect ratio of cold storage material paricle | Maximum volumetric specific heat value at 10 K or less | Thermal conductivity at 4.2 K | Refrigeration capacity at 4.2 K |
|---|---|---|---|---|---|---|---|---|---|---|---|---|---|
| | | | | (atom %) | | (atom %) | | (atom% ) | (µm) | | (J/cm³·K) | (W/cm. K) | (W) |
| Ex ample 60 | Rolling granulation | GdAlO3 | Na | 0.55% | | | Zr | 15.00% | 250 | 1 | 0.8 | 0.015 | 0.66 |
| Example 61 | Rolling granulation | GdAlO3 | Na | 0.59% | Ca | 0.004% | Zr | 15.00% | 440 | 1.1 | 0.8 | 0.02 | 0.67 |
| Ex ample 62 | Syringe dropping | GdAlO3 | Na | 0.83% | Ca | 0.16% | Zr | 0.01% | 330 | 1.1 | 1.2 | 0.02 | 0.72 |
| Comparative Example 9 | Rolling granulation | GdAlO3 | Na | 0.55% | | | Zr | 25.00% | 250 | 1 | 0.4 | 0.02 | 0.3 |

As in Comparative Example 1 and Comparative Example 4, it can be seen that, when the atomic concentration of the Group 1 element in the cold storage material is less than 0.001 atom% or the cold storage material does not contain any Group 1 element, the thermal conductivity is as low as 0.005 W/cm·K and 0.004 W/(cm·K). This is considered to be because the sintering promoting effect was reduced as the proportion of the Group 1 element in the cold storage material decreased, resulting in an increase in the amount of fine voids.

As in Comparative Example 2, it can be seen that, when the atomic concentration of the Group 1 element in the cold storage material is more than 10 atom%, the volumetric specific heat is as low as 0.4 J/(cm³·K). This is considered to be because an oxide containing a rare earth and a Group 1 element was generated as the proportion of the Group 1 element in the cold storage material increased, resulting in a decrease in the relative amount of the rare earth aluminum oxide.

From the results of Comparative Example 3, when the atomic concentration of the Group 2 element in the cold storage material was more than 10 atom%, the thermal conductivity was high, but the volumetric specific heat was as low as 0.4 J/(cm³·K), and the refrigeration capacity using these cold storage material particles significantly decreased. This is considered to be because the proportion of the rare earth element relatively decreased as the proportion of the Group 2 element in the cold storage material particles increased.

From the results of Tables 1 and 4, the strength was increased by adding the sintering aid so that the amount of the metal or metalloid element derived from the sintering aid was 0.01 atom% or more. When the amount of the metal or metalloid element derived from the sintering aid was more than 20 atom%, the strength was further increased, but the volumetric specific heat was as low as 0.4 J/(cm³·K), and the refrigeration capacity using these cold storage material particles significantly decreased. This is considered to be because the proportion of the rare earth element relatively decreased as the proportion of the sintering aid in the cold storage material particles increased.

From Tables 1 and 2, it can be seen that, when the particle diameter of the cold storage material particles falls within the range of 50 µm or more and 3000 µm or less, the refrigeration capacity at 4.2 K is remarkably improved.

Tables 1 and 2 show that, when the aspect ratio of the cold storage material particles is 5 or less, the refrigeration capacity at 4.2 K is remarkably improved.

From Tables 1 and 2, it can be seen that, even if the methods of granulating cold storage material particles are different, the cold storage material particles have the same particle diameter, the same aspect ratio, the same Groups 1 and 2 element contents, and the same thermal conductivity by appropriately adjusting the synthesis conditions.

From Tables 1 and 2, it can be seen that, even if the methods of granulating cold storage material particles are different, refrigerators filled with the cold storage material particles have the same performance and the same reliability as long as they have the same particle diameter, the same aspect ratio, and the same thermal conductivity.

From Table 3, it can be seen that the strength of the granulated particles is high when the Group 1 element is contained in an amount of 0.001 atom% or more and 10 atom% or less, and the carbon is contained in an amount of 0.01 wt% or more and 20 wt% or less before the granulated particles are degreased. If the carbon concentration is lower than 0.01 wt%, the granulated particles are very fragile and cannot be recovered as particles.

From Table 3, it can be seen that both the strength and the sinterability of the granulated particles can be achieved when the Group 1 element is contained in an amount of 0.001 atom% or more and 10 atom% or less, and carbon is contained in an amount of 0.001 wt% or more and 10 wt% or less after the granulated particles are degreased. In addition, a refrigerator filled with the obtained cold storage material particles exhibits a high refrigeration performance. When the carbon concentration after the degreasing exceeds 10 wt%, the strength of the particles is maintained even after the degreasing, but the molding density is low and the sinterability is poor, the density of the particles is low, and the specific heat and the thermal conductivity are low. Accordingly, a refrigerator filled with the cold storage material particles has a low refrigeration performance. If the carbon concentration is lower than 0.001 wt%, the particles after being degreased are very fragile and cannot be recovered as particles.

From the above-described examples, the effects exhibited by the cold storage material according to the first embodiment and the cold storage material particles according to the second embodiment were confirmed.

Although the air pulse type dispenser or the piezo type dispenser has been described as an example of the dispenser, a plunger type dispenser may be used.

Although the continuous type inkjet has been described as an example of the inkjet, an on-demand type inkjet may be used.

Although some embodiments of the present invention have been described, these embodiments are exemplary, and are not intended to limit the scope of the invention. These novel embodiments can be implemented in various other forms, and various omissions, substitutions, and changes can be made without departing from the gist of the invention. For example, a component of one embodiment may be replaced or changed with a component of another embodiment. These embodiments and modifications thereof fall within the scope and gist of the invention, and fall within the scope of the equivalent to the invention set forth in the claims.

### Reference Signs List

100 Cold storage cryogenic refrigerator
114, 115 Cold storage device
118, 119 Cold storage material
500 Cryopump
600 Superconducting magnet
700 Nuclear magnetic resonance imaging apparatus
800 Nuclear magnetic resonance apparatus
900 Magnetic field application type single crystal pulling apparatus
1000 Helium re-condensing device
1100 Dilution refrigerator

## Claims

1. A cold storage material comprising:
a rare earth aluminum oxide containing at least one rare earth element selected from the group consisting of Y, La, Ce, Pr, Nd, Sm, Eu, Gd, Tb, Dy, Ho, Er, Tm, Yb, and Lu; and
a Group 1 element in an amount of 0.001 atom% or more and 10 atom% or less,
wherein a maximum volumetric specific heat value in a temperature range of 2 K or more and 10 K or less is 0.5 J/ (cm³·K) or more.

2. The cold storage material according to claim 1, wherein the Group 1 element is at least one element selected from the group consisting of Li, Na, and K.

3. The cold storage material according to claim 1, further comprising a Group 2 element in an amount of 0 atom% or more and 10 atom% or less.

4. The cold storage material according to claim 1, further comprising a Group 2 element in an amount of 0.001 atom% or more and 10 atom% or less.

5. The cold storage material according to claim 3, wherein the Group 2 element is at least one element selected from the group consisting of Mg, Ca, Sr, and Ba.

6. The cold storage material according to claim 1, further comprising at least one element selected from the group consisting of Fe, Cu, Ni, Co, Zr, and B in an amount of 0.01 atom% or more and 20 atom% or less.

7. A cold storage material particle made of the cold storage material according to claim 1, wherein the cold storage material particle has a particle diameter of 50 µm or more and 3 mm or less.

8. The cold storage material particle according to claim 7, wherein the cold storage material particle has an aspect ratio of 1 or more and 5 or less.

9. A granulated particle that is a raw material of the cold storage material particle according to claim 7, the granulated particle comprising:
a rare earth oxide containing at least one rare earth element selected from the group consisting of Y, La, Ce, Pr, Nd, Sm, Eu, Gd, Tb, Dy, Ho, Er, Tm, Yb, and Lu;
an aluminum oxide;
the Group 1 element in an amount of 0.001 atom% or more and 10 atom% or less; and
carbon in an amount of 0.01 wt% or more and 20 wt% or less,
wherein the granulated particle has a particle diameter of 70 µm or more and 5 mm or less and an aspect ratio of 1 or more and 5 or less.

10. A granulated particle after being degreased that is a raw material of the cold storage material particle according to claim 7, the granulated particle comprising:
a rare earth oxide containing at least one rare earth element selected from the group consisting of Y, La, Ce, Pr, Nd, Sm, Eu, Gd, Tb, Dy, Ho, Er, Tm, Yb, and Lu;
an aluminum oxide;
the Group 1 element in an amount of 0.001 atom% or
more and 10 atom% or less; and
carbon in an amount of 0.001 wt% or more and 10 wt% or less,
wherein the granulated particle after being degreased has a particle diameter of 70 µm or more and 5 mm or less and an aspect ratio of 1 or more and 5 or less.

11. A cold storage device comprising the cold storage material according to claim 1.

12. A cold storage device filled with a plurality of the cold storage material particles according to claim 7.

13. A refrigerator comprising the cold storage device according to claim 11 or 12.

14. A cryopump comprising the refrigerator according to claim 13.

15. A superconducting magnet comprising the refrigerator according to claim 13.

16. A nuclear magnetic resonance imaging apparatus comprising the refrigerator according to claim 13.

17. A nuclear magnetic resonance apparatus comprising the refrigerator according to claim 13.

18. A magnetic field application type single crystal pulling apparatus comprising the refrigerator according to claim 13.

19. A helium re-condensing device comprising the refrigerator according to claim 13.

20. A dilution refrigerator comprising the refrigerator according to claim 13.
